(19) Europäisches Patentamt
European Patent Office
Office européen des brevets

(11) **EP 3 785 601 B1**

(12) **EUROPÄISCHE PATENTSCHRIFT**

(45) Veröffentlichungstag und Bekanntmachung des Hinweises auf die Patenterteilung:
**18.10.2023 Patentblatt 2023/42**

(21) Anmeldenummer: **20186735.5**

(22) Anmeldetag: **13.12.2012**

(51) Internationale Patentklassifikation (IPC):
**A61B 3/11** (2006.01) **G02C 7/02** (2006.01)
**G02C 7/06** (2006.01)

(52) Gemeinsame Patentklassifikation (CPC):
**G02C 7/027; A61B 3/11; A61B 3/112; G02C 7/02; G02C 7/066**

(54) **HELLIGKEITSABHÄNGIGE ANPASSUNG EINES BRILLENGLASES**

BRIGHTNESS-DEPENDENT ADJUSTMENT OF A SPECTACLE LENS

AJUSTEMENT EN FONCTION DE LA LUMINOSITÉ D'UN VERRE DE LUNETTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011120974**

(43) Veröffentlichungstag der Anmeldung:
**03.03.2021 Patentblatt 2021/09**

(62) Dokumentnummer(n) der früheren Anmeldung(en) nach Art. 76 EPÜ:
**12813758.5 / 2 790 567**

(73) Patentinhaber: **Rodenstock GmbH**
**80687 München (DE)**

(72) Erfinder:
• **TRUMM, Stephan**
**80999 München (DE)**
• **SEITZ, Peter**
**71272 Renningen (DE)**
• **SESSNER, Rainer**
**91154 Roth (DE)**
• **MUSCHIELOK, Adam**
**81476 München (DE)**
• **ALTHEIMER, Helmut**
**87650 Baisweil-Lauchdorf (DE)**
• **BECKEN, Wolfgang**
**82061 Neuried (DE)**
• **WELK, Andrea**
**81547 München (DE)**
• **SEIDEMANN, Anne**
**81375 München (DE)**
• **HAUK, Wolfgang**
**86156 Augsburg (DE)**
• **ESSER, Gregor**
**80686 München (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A1-2004/072709    WO-A1-2006/116820**
**WO-A2-2007/137100    US-A1- 2011 001 925**

**Beschreibung**

Die vorliegende Erfindung betrifft die Optimierung und Herstellung von Brillengläsern für einen Brillenträger unter Berücksichtigung von Lichtverhältnissen einer individuellen Gebrauchssituation, für welche das jeweilige Brillenglas optimiert werden soll.

Für die Herstellung bzw. Optimierung von Brillengläsern, insbesondere von individuellen Brillengläsern wird jedes Brillenglas so gefertigt, dass für jede gewünschte Blickrichtung oder jeden gewünschten Objektpunkt eine möglichst gute Korrektur eines Refraktionsfehlers des jeweiligen Auges des Brillenträgers erreicht wird. Im Allgemeinen gilt ein Brillenglas für eine gegebene Blickrichtung dann als vollkorrigierend, wenn die Werte Sphäre, Zylinder und Achse der Wellenfront beim Passieren der Scheitelpunktkugel mit den Werten für Sphäre, Zylinder und Achse der Verordnung für das fehlsichtige Auge übereinstimmen. Bei der Refraktionsbestimmung für ein Auge eines Brillenträgers werden dioptrische Werte (insbesondere Sphäre, Zylinder, Achslage) für eine weite (i.d.R. unendliche) Entfernung und gegebenenfalls (für Mehrstärkengläser bzw. Gleitsichtgläser) eine Addition für eine nahe Entfernung (z.B. nach DIN 58208) bestimmt. Damit ist die Verordnung (insbesondere Sphäre, Zylinder, Achslage und gegebenenfalls Addition) festgelegt, die an einen Brillenglashersteller übermittelt wird. Bei modernen Brillengläsern können zusätzlich auch von der Norm abweichende Objektentfernungen, die bei der Refraktionsbestimmung verwendet wurden, angegeben werden.

Eine vollständige Korrektur für alle Blickrichtungen gleichzeitig ist aber im Normalfall nicht möglich. Daher werden die Brillengläser derart gefertigt, dass sie vor allem in den hauptsächlichen Nutzungsbereichen, insbesondere in den zentralen Durchblicksbereichen eine gute Korrektur von Fehlsichtigkeiten des Auges und nur geringe Abbildungsfehler bewirken, während in peripheren Bereichen größere Abbildungsfehler zugelassen werden.

Um ein Brillenglas derart fertigen zu können, erfolgt zunächst eine Berechnung der Brillenglasflächen bzw. zumindest einer der Brillenglasflächen derart, dass dadurch die gewünschte Verteilung der unvermeidlichen Abbildungsfehler bewirkt wird. Diese Berechnung und Optimierung erfolgt üblicherweise mittels eines iterativen Variationsverfahren durch Minimieren einer Zielfunktion. Als Zielfunktion wird insbesondere eine Funktion F mit folgendem funktionalen Zusammenhang zur sphärischen Wirkung S, zum Betrag der zylindrischen Wirkung Z und zur Achslage des Zylinders $\alpha$ (auch als "SZA"-Kombination bezeichnet) berücksichtigt und minimiert:

$$F = \sum_{i=1}^{m} \left[ g_{i,S\Delta} \left( S_{\Delta,i} - S_{\Delta,i,Soll} \right)^2 + g_{i,Z\Delta} \left( Z_{\Delta,i} - Z_{\Delta,i,Soll} \right)^2 + ... \right] \ .$$

Dabei werden in der Zielfunktion F an den Bewertungsstellen / des Brillenglases zumindest die tatsächlichen Refraktionsdefizite der sphärischen Wirkung $S_{\Delta,i}$ und der zylindrischen Wirkung $Z_{\Delta,i}$ sowie Sollvorgaben für die Refraktionsdefizite der sphärischen Wirkung $S_{\Delta,i,Soll}$ und der zylindrischen Wirkung $Z_{\Delta,i,Soll}$ berücksichtigt.

Bereits in DE 103 13 275 wurde erkannt, dass es vorteilhaft ist, die Sollvorgaben nicht als absolute Werte der zu optimierenden Eigenschaften sondern als deren Abweichung von der Verordnung, also als geforderte Fehlanpassung anzugeben. Dies hat den Vorteil, dass die Sollvorgaben unabhängig von der Verordnung ($Sph_V, Zyl_V, Achse_V, Pr_V, B_V$) sind und die Sollvorgaben nicht für jede individuelle Verordnung geändert werden müssen. Als "Ist"-Werte der zu optimierenden Eigenschaften fließen in die Zielfunktion somit auch nicht absolute Werte dieser optischen Eigenschaften, sondern die Abweichungen von der Verordnung ein. Dies hat den Vorteil, dass die Sollvorgaben unabhängig von der Verordnung vorgegeben werden können und nicht für jede individuelle Verordnung geändert werden müssen.

Die jeweiligen Refraktionsdefizite an den jeweiligen Bewertungsstellen werden vorzugsweise mit Gewichtungsfaktoren $g_{i,S\Delta}$ bzw. $g_{i,Z\Delta}$ berücksichtigt. Dabei bilden die Sollvorgaben für die Refraktionsdefizite der sphärischen Wirkung $S_{\Delta,i,Soll}$ und/oder der zylindrischen Wirkung $Z_{\Delta,i,Soll}$ insbesondere zusammen mit den Gewichtungsfaktoren $g_{i,S\Delta}$ bzw. $g_{i,Z\Delta}$ das sogenannte "Design" oder Brillenglasdesign. Darüber hinaus können insbesondere auch weitere Residuen, insbesondere weitere zu optimierende Größen, wie z.B. Koma und/oder sphärische Aberration und/oder Prisma und/oder Vergrößerung und/oder anamorphotische Verzerrung, usw., berücksichtigt werden, was insbesondere durch den Ausdruck "+..." angedeutet ist. Das Design eines Brillenglases legt somit insbesondere die Art der Verteilung der Abbildungsfehler auf dem Brillenglas fest. Vorzugsweise wird für eine Vielzahl von Bewertungspunkten auf dem Brillenglas festgelegt wie groß der zu erreichende Abbildungsfehler und gegebenenfalls dessen Gewichtung in der Zielfunktion sein soll.

Vorzugsweise umfasst das Design eines Brillenglases auch eine Festlegung der Position eines oder mehrerer ausgezeichneter Punkte, insbesondere Bezugspunkte, wie z.B. die Position eines Fernbezugspunktes und/oder eines Nahbezugspunktes und/oder eines Prismenbezugspunktes und/oder eines Zentrierpunktes und/oder eine Position oder ein Verlauf einer Hauptblicklinie. Während Brillenglasdesigns in den Anfängen der progressiven Gläser vom Brillenglashersteller nach verschiedenen Kriterien abgestuft vorgegeben wurden, wurde die Anpassung von Brillengläsern für einzelne Brillenträger im Laufe der Jahre immer mehr individualisiert. Insbesondere wurden dabei die Brillenglasdesigns an individuelle Gegebenheiten, wie z.B. Maße und Position der individuellen Brillenfassung (z.B. Vorneigung, Fassungsscheibenwinkel), habituelle Parameter (z.B. Kopfhaltung, Kopfbewegung), eine individuelle Gebrauchssituation (individuelles Objekt-Abstands-Modell) oder individuelle anatomische Gegebenheiten (z.B. Hornhaut-Scheitel-Abstand) berücksichtigt.

Neben dem Design des Brillenglases spielt auch die korrekte Zentrierung eine wichtige Rolle. So werden zur Anpassung von Brillen derzeit - neben anderen Parametern - hauptsächlich die Pupillendistanzen beim Blick in die Ferne (Fern-PD) gemessen. Im Fall von Gleitsichtbrillen wird üblicherweise - auch bei ansonsten hoch individualisierten Gläsern - die Fern-PD gemessen und zur Ermittlung der Nah-PD bzw. des Verlaufes der Hauptblicklinie mit einem vereinfachten Modell (Drehung des Auges um einen Punkt) und Standardparametern (üblicherweise der Augenradius aus dem Modell nach Gullstrand, siehe DIN 5340) gearbeitet. Gemessen wird die Fern-PD mit den meisten herkömmlichen Videozentriersystemen näherungsweise, indem eine Sehaufgabe für einen gewissen Abstand zum Gerät gestellt wird, der als ausreichend groß gilt.

Um eine individuelle Anpassung eines Brillenglases bzw. einer Brille weiter zu verbessern, wurden auch deutlich detailliertere Modelle zur Beschreibung der Anatomie des Auges und des das Auge bewegenden Muskelapparats in Betracht gezogen. So lassen sich für horizontale und vertikale Rotationen unterschiedliche Achsen mit verschiedenen Radien festlegen. Ferner lässt sich zwischen dem mechanischen und dem optischen Augendrehpunkt unterscheiden. Trotz aller bisherigen Bemühungen um eine möglichst genaue Erfassung der individuellen Augenbewegung weist die Qualität der individuellen Anpassung eines Brillenglases bzw. einer Brille stets ihre Grenzen auf.

Zur Anpassung von Brillen werden bisher neben anderen Parametern insbesondere die Position der Pupille (Pupillendistanzen und Einschleifhöhen) beim Blick in die Ferne unter im Allgemeinen undefinierten Lichtverhältnissen manuell oder mittels Videozentriersystemen gemessen. Eine Betrachtung der tatsächlichen Größen oder Positionen der Pupillen für die beim späteren Gebrauch der Brille vorliegenden Lichtverhältnisse findet dabei nicht statt.

WO 2004/072709 A1 beschreibt ein Verfahren, bei dem der visuelle Effekt verschiedener Aberrationen höherer Ordnung in Bezug auf den durch Defokussierung erzeugten visuellen Effekt normalisiert ist. Ferner ist ein Korrekturfaktor, der RMS normalisiert, ein Testdiagramm zum Messen der Effekte von Aberrationen höherer Ordnung, Verfahren zum Testen der Effekte von Aberrationen höherer Ordnung, Modelle zur Ermittlung des relativen visuellen Effekts von Aberrationen, ein Verfahren zum Entwerfen von Kontaktlinsen, ein Verfahren, bei dem die VPDF verwendet wird, um das Design sowohl der Vorder- als auch der Rückseite der Linse zu optimieren, Verfahren zum Entwerfen eines chirurgischen Verfahrens und Verfahren der Augenchirurgie beschrieben.

US 2011/001925 A1 beschreibt ein Verfahren zur Herstellung einer progressiven Brille für einen presbyopen Träger, die die Haltungsgewohnheiten des Trägers berücksichtigt, die durch vorherige Bedingungen von Augenkorrektur erworben wurden. Ein Design für die progressive Brille wird erstellt, indem eine Referenzbrille entsprechend der Eignung des Trägers modifiziert wird, seine Haltungsgewohnheiten zu ändern. Dazu werden Variationen eines Haltungsparameters anhand von Messungen dieses Parameters bestimmt, die am Träger durchgeführt werden.

WO 2006/116820 A1 beschreibt eine Anordnung progressiver ophthalmischer Linsenelemente. Die progressiven ophthalmischen Elemente, die in der Anordnung enthalten sind, haben im Wesentlichen die gleiche Additionsstärke und im Wesentlichen die gleiche optische Verordnung für die Fernsicht. Jedes der progressiven ophthalmischen Linsenelemente weist ein progressives Linsendesign auf, das durch einen Satz von Parametern gekennzeichnet ist, die eine Fernzone definieren, die eine Brechkraft für die Fernsicht bereitstellt, eine Nahzone, die eine Brechkraft für die Nahsicht bereitstellt, und einen Korridor mit einer Brechkraft variierend von der der Fernzone zu der der Nahzone. Die progressiven ophthalmischen Elemente stellen für eine Reihe von Werten oder Kategorien von mindestens zwei Lebensstil- und/oder biometrischen Parametern von Brillenträgern unterschiedliche progressive Linsendesigns bereit, bei denen mindestens zwei der Brillenglasdesignparameter jeweils einen entsprechenden Wert oder Eigenschaft aufweisen, welcher bzw. welche einem bestimmten Wert oder einer Kategorie eines jeweiligen der Lebensstil- und/oder biometrischen Parameter zuordenbar oder damit verbindbar sind.

Aufgabe der vorliegenden Erfindung ist es, eine Verbesserung der individuellen Anpassung von Brillengläsern zu erreichen. Diese Aufgabe wird wie in dem unabhängigen Anspruch 1 angegeben gelöst. Die Erfindung ist durch den unabhängigen Anspruch definiert, während bevorzugte Ausführungsformen Gegenstand der abhängigen Ansprüche sind.

Somit bietet die Erfindung in einem Aspekt ein Verfahren zum Anpassen, insbesondere Optimieren und Herstellen, eines individuellen Brillenglases für zumindest ein Auge eines Brillenträgers, umfassend insbesondere ein Erfassen von individuellen Daten für das zumindest eine Auge des Brillenträgers. Dabei umfasst das Erfassen von individuellen Daten ein Festlegen einer individuellen Gebrauchssituation, welche zumindest einen Helligkeitssollwert für das von dem zumindest einen Auge in der geplanten Gebrauchssituation, in welcher das Brillenglas zum Einsatz kommen soll, zu erfassende Licht umfasst. Als Helligkeitssollwert wird vorzugsweise ein Sollwert der (mittleren) Leuchtdichte festgelegt. Vorzugsweise legt die individuelle Gebrauchssituation auch ein Objekt-Abstands-Modell zumindest teilweise fest, d.h. es legt zumindest für eine oder einige Blickrichtungen die in der individuellen Gebrauchssituation eines anzupassenden Brillenglases erwartete Entfernung von Objekten fest. Für ein Gleitsichtglas werden dabei beispielsweise zumindest die erwartete Objektentfernung für eine einem Fernbezugspunkt des Brillenglases entsprechende Blickrichtung und eine einem Nahbezugspunkt des Brillenglases entsprechende Blickrichtung festgelegt.

Außerdem umfasst das Verfahren (insbesondere das Erfassen von individuellen Daten) ein Bestimmen einer bei dem zumindest einen Helligkeitssollwert auftretenden oder erwarteten individuellen Position der Pupille des zumindest einen Auges bei zumindest einer Blickrichtung des zumindest einen Auges, also bei zumindest einer Augenstellung. Es wird also die individuelle Position der Pupille bestimmt, die sich für das zumindest eine Auge des Brillenträgers bei dem in der individuellen Gebrauchssituation festgelegten Sollwert der vom Auge erfassten (mittleren) Leuchtdichte ergibt. Dabei werden insbesondere zwei alternative Vorgehensweisen bevorzugt.

In einer dieser bevorzugten Vorgehensweisen wird die individuelle Position der Pupille (direkt oder indirekt) erfasst, während das zumindest eine Auge dem festgelegten Sollwert der Helligkeit ausgesetzt ist. Es wird also während der Erfassung der Pupillenposition in der zumindest einen Blickrichtung der in der späteren tatsächlichen Gebrauchssituation erwartete Helligkeitszustand eingestellt.

In einer anderen bevorzugten Vorgehensweise wird ein formeller Zusammenhang zwischen der Helligkeit und der Position der Pupille des zumindest einen Auges festgelegt. Außerdem wird die während der Erfassung der Position der Pupille auf das Auge wirkende Helligkeit erfasst. Anschließend wird zur Transformation auf den Helligkeitssollwert mittels des festgelegten Zusammenhangs zwischen der Helligkeit und der Position eine entsprechende Korrektur der Position der Pupille vorgenommen.

Das Festlegen des zumindest einen Helligkeitssollwertes erfolgt vorzugsweise durch eine Benutzereingabe, entweder direkt als Zahlenwert oder durch Auswahl aus vorgegebenen Zahlenwerten oder aus einem vorgegebenen Wertebereich, oder durch Auswahl aus vorgegebenen Anwendungsfeldern, für welche typische Helligkeitswerte hinterlegt sind (z.B. Unterscheidung zwischen Tag- und Nacht-Brillen für Autofahrer; Sport im Freien, Arbeiten am Computer, usw.). Bisher wurden solche Gebrauchssituationen zwar gelegentlich in den typischen Objektabständen unterschieden, eine Berücksichtigung der im Einzelfall zu erwartenden Helligkeiten und deren individuellen Einfluss auf die Position der Pupille fand aber nicht statt. So wurde auch bei der Vermessung der Augen insbesondere für eine Zentrierung bisher nicht auf die tatsächlich während der Messung herrschende Helligkeit geachtet.

In der vorliegenden Erfindung hingegen wurde erkannt, dass durch die Berücksichtigung der tatsächlichen Helligkeit in der individuellen Gebrauchssituation und deren individuellen Einflusses auf die Position der Pupille, insbesondere bei einer asymmetrischen Adaption der Pupille für verschiedene Helligkeiten, allein schon beim Zentrieren eines Brillenglases, vorzugsweise aber auch beim Optimieren und Herstellen eines Brillenglases in einfacher Weise eine Verbesserung in der individuellen Anpassung von Brillengläser erreicht wird.

Dabei umfasst das Verfahren außerdem ein Bestimmen eines Referenzpunktes des Brillenglases, in dem das Brillenglas eine für die zumindest eine Blickrichtung geforderte Korrektur von individuellen Refraktionsdaten bewirkt. Die Forderung der Korrektur wird vorzugsweise durch eine Zielfunktion festgelegt, in die individuell ermittelte Refraktionsdaten, insbesondere für verschiedene Blickrichtungen (z.B. als Brechwertverlauf), zusammen mit Toleranzen bzw. Vorgaben für Abweichungen von der vollständigen Korrektur (dem sogenannten Design) einfließen. Je nach geplanter Anwendung werden die Refraktionsdaten somit vorzugsweise sogar bei einer Vielzahl von Blickrichtungen erfasst. So werden für ein Mehrstärkenglas oder ein Gleitsichtglas vorzugsweise zumindest eine Fernrefraktion und eine Nahrefraktion des zumindest einen Auges erfasst, woraus sich für ein Gleitsichtglas insbesondere auch die erforderliche Addition ergibt.

Die erforderliche optische Wirkung des zu optimierenden und herzustellenden Brillenglases hängt dabei für jede Blickrichtung insbesondere von der Entfernung der Objekte in der geplanten Gebrauchssituation ab. Diese wird vorzugsweise für jede Blickrichtung durch das vorgegebene Objekt-Abstands-Modell beschrieben. Als zumindest ein Referenzpunkt kann dabei beispielsweise ein Fernbezugspunkt und/oder ein Nahbezugspunkt und/oder ein Zentrierpunkt des Brillenglases gewählt werden. Die in dem zumindest einen Referenzpunkt zumindest teilweise zu korrigierende individuelle Refraktion kann dabei entweder direkt für den Brillenträger gemessen werden (z.B. für den Fern- oder Nahbezugspunkt) oder aus gemessenen Werten für andere Blickrichtungen z.B. aus einem gewünschten Verlauf eines Wirkungsanstiegs zwischen Fern- und Nahbereich abgeleitet werden (z.B. für einen vom Fern- oder Nahbezugspunkt abweichenden Referenzpunkt).

Außerdem umfasst das Verfahren ein Bereitstellen und Anordnen des Brillenglases (insbesondere ein Einschleifen des Brillenglases in die Fassung) derart, dass der zumindest eine Referenzpunkt des Brillenglases (z.B. der Zentrierpunkt) in Abhängigkeit von dem bestimmten individuellen Wert der Position der Pupille vor dem zumindest einen Auge des Brillenträgers angeordnet wird. Insbesondere wird also mit anderen Worten das Brillenglas derart bereitgestellt und angeordnet (bzw. zur Anordnung ausgestaltet), dass der Brillenträger in der individuellen Gebrauchssituation beim Blicken in die zumindest eine Blickrichtung, für welche der individuelle Wert der Position der Pupille (also die individuelle Position der Pupille) bestimmt wurde, durch den bestimmten Referenzpunkt des Brillenglases blickt. So wird vorzugsweise ein als Referenzpunkt herangezogener Zentrierpunkt des Brillenglases insbesondere beim Blick in der zumindest einen Blickrichtung horizontal vor der bei dem festgelegten Helligkeitssollwert zu erwartenden bzw. gegebenen Position der Pupille angeordnet. Es erfolgt also insbesondere für das Einschleifen des Glases in die gewünschte Brillenfassung eine von der in der individuellen Gebrauchssituation auftretenden Helligkeit abhängige Zentrierung des Brillenglases.

Während bisher beispielsweise für die Zentrierung des Brillenglases weder beim Vermessen der Zentrierdaten (z.B. der Pupillendistanz oder der Einschleifhöhe) auf die in diesem Moment herrschende Helligkeit noch auf die Helligkeit für die gewünschte Anwendungssituation geachtet wurde, erfolgt erfindungsgemäß eine individuelle helligkeitsabhängige Zentrierung, indem der individuelle Einfluss der Helligkeit auf die Pupillenposition ermittelt und berücksichtigt wird.

Unter Zentrierung wird dabei im Folgenden nicht nur die Positionierung der Brillengläser in der Fassung sondern vorzugsweise auch die Lage der Designpunkte zueinander und vorzugsweise sogar der Verlauf der Hauptblicklinie verstanden.

So erfolgt in einem weiteren Aspekt der Erfindung sogar eine Berücksichtigung der individuellen helligkeitsabhängigen Position der Pupille bei der Optimierung eines Brillenglases. In diesem Aspekt bietet die Erfindung somit ein Verfahren zum Optimieren und Herstellen eines individuellen Brillenglases für zumindest ein Auge eines Brillenträgers, umfassend ein Festlegen einer individuellen Gebrauchssituation, welche für zumindest zwei verschiedene Referenzpunkte des Brillenglases jeweils einen Helligkeitssollwert für das von dem zumindest einen Auge zu erfassende Licht festlegt. Gebrauchsdaten, welche die individuelle Gebrauchssituation beschreiben, umfassen jeweils einen Wert für zu erwartenden Helligkeiten. Damit erfolgt also eine helligkeitsabhängige Berücksichtigung der Position der Pupille für verschiedene Blickrichtungen bei der Optimierung und Herstellung eines Brillenglases. Dabei kann die in der individuellen Gebrauchssituation erwartete (festgelegte) Helligkeit durchaus für alle Blickrichtungen (insbesondere für die beiden Referenzpunkte) die gleiche sein. Damit reicht es vorzugsweise aus, einen einzigen Helligkeitssollwert festzulegen, der dann auf alle Referenzpunkte anwendbar ist.

Außerdem umfasst das Verfahren ein Bestimmen von Refraktionsdaten des zumindest einen Auges für die zumindest zwei verschiedenen Referenzpunkte und damit insbesondere für eine Vielzahl von Objektentfernungen, insbesondere für zumindest zwei Sehbereiche bzw. Blickrichtungen, welche insbesondere einer Fernsicht und einer Nahsicht entsprechen.

Als Referenzpunkte insbesondere eines Gleitsichtglases könnten dabei vorzugsweise ein Fernbezugspunkt und ein Nahbezugspunkt vorgesehen werden. Dabei werden für den Fern- und Nahbezugspunkt insbesondere verschiedene Helligkeitssollwerte in der individuellen Gebrauchssituation festgelegt. Die im Fern- und Nahbezugspunkt auftretende unterschiedliche Helligkeit in der individuellen Gebrauchssituation kann individuell zu unterschiedlichen Positionen und/oder Größen der Pupille führen. Um dies bei der Optimierung und Herstellung des Brillenglases zu berücksichtigen, werden insbesondere diese Bezugspunkte gemäß der individuellen, helligkeitsabhängigen Pupillenposition angepasst, also insbesondere ausgehend von einem Solldesign (Startdesign), welches ohne Berücksichtigung einer Abhängigkeit der Pupillenposition von der Helligkeit erstellt wurde, verschoben.

Die Unterscheidung zwischen Fern- und Nahsicht ist insbesondere bei progressiven Brillengläser wünschenswert. In diesem Fall ergeben sich für die zumindest zwei Referenzpunkte vorzugsweise unterschiedliche Refraktionsdaten. Die Berücksichtigung des individuellen Einflusses der Helligkeit auf die Position der Pupille bei der Optimierung ist aber auch für Referenzpunkte vorteilhaft, in denen gleiche Refraktionsdaten des Auges zu korrigieren sind. So bietet die erfindungsgemäße Vorgehensweise insbesondere auch für individuell berechnete (optimierte) Einstärkengläser eine Verbesserung der individuellen Anpassung.

Dazu umfasst das Verfahren ein Bestimmen eines individuellen Einflusses der Helligkeit des von dem zumindest einen Auge erfassten Lichts auf die Position der Pupille des zumindest einen Auges und ein Optimieren und Herstellen des Brillenglases, welches eine für die Referenzpunkte bestimmte Korrektur der Refraktionsdaten bei der sich aus dem bestimmten Einfluss der Helligkeit auf die Position der Pupille für die für die Referenzpunkte festgelegten Helligkeitssollwerte ergebenen Positionen der Pupille des zumindest einen Auges bewirkt.

Im Rahmen der vorliegenden Erfindung wurde also erkannt, dass eine Verbesserung der individuellen Anpassung einer Brille oder eines Brillenglases dadurch erreicht wird, dass eine helligkeitsabhängige Zentrierung und/oder Optimierung in einer Weise vorgenommen wird, in der insbesondere individuelle nicht symmetrische Veränderungen einer Pupille bei einer Anpassung an die Helligkeit (Adaption) zumindest teilweise Berücksichtigung finden.

Vorzugsweise umfasst das Optimieren des Brillenglases ein Minimieren einer Zielfunktion, insbesondere gemäß der oben genannten Zielfunktion F. Besonders bevorzugt umfasst das Verfahren ein Bestimmen einer jeweils bei den festgelegten Helligkeitssollwerten auftretenden oder erwarteten individuellen Position und/oder Größe der Pupille, wobei das Optimieren des Brillenglases ein Minimieren einer Zielfunktion umfasst, welche für die zumindest zwei Referenzpunkte eine vom Brillenglas in einer Umgebung des jeweiligen Referenzpunktes bewirkten Korrektur der jeweils für den jeweiligen Referenzpunkt bestimmten Refraktionsdaten bewertet, wobei insbesondere die Größe der Umgebung des jeweiligen Referenzpunktes in Abhängigkeit von der für den jeweiligen Referenzpunkt bestimmten individuellen Größe der Pupille gewählt wird. So erfolgt eine Auswertung der Zielfunktion vorzugsweise in bekannter Weise auf der Scheitelpunktkugel eines das System aus Objekt, Brillenglas und Auge beschreibenden Modells. Dabei wird die Wirkung des Brillenglases vorzugsweise mittels Ray-Tracing und/oder Wavefront-Tracing, also insbesondere durch Strahl- und/oder Wellenfront-Durchrechnung ausgehend von einem Objektpunkt durch das Brillenglas bis hin zur Scheitelpunktkugel berechnet und in der Zielfunktion mit dem auf die Scheitelpunktkugel projizierten Refraktionsfehler des Auges kombiniert. Besonders bevorzugt erfolgt dabei die Auswertung der Wellenfront zu einem durch den jeweiligen Referenzpunkt verlaufenden Hauptstrahl in einer von der entsprechenden Pupillengröße abhängigen Umgebung des Hauptstrahls. Damit werden insbesondere Abbildungsfehler höherer Ordnung besonders gut der realen Gebrauchssituation entsprechend korrigiert. Mögliche Vorgehensweisen zur Berücksichtigung von Abbildungsfehlern höherer Ordnung insbesondere gemäß weiter bevorzugter Ausführungsformen der vorliegenden Erfindung werden weiter unten noch ausführlich beschrieben. So können Abbildungsfehler höherer Ordnung beispielsweise mittels Aberrometer individuell gemessen werden. In einer anderen, weiten unten ausführlich beschriebenen, bevorzugten Ausführungsform können solche Abbildungsfehler insbesondere in Abhängigkeit von anderen individuell ermittelten Parametern aus statistischen Daten gewonnen werden.

Die individuelle Position der Pupille für die Gebrauchssituation wird dabei in einer bevorzugten Ausführungsform dadurch berücksichtigt, dass das Verfahren umfasst: Ermitteln, insbesondere Messen jeweils einer Position eines Referenzpunktes des Auges für Blickrichtungen, welche den zumindest zwei Referenzpunkten des Brillenglases entsprechen, also für Blickrichtungen durch den jeweiligen Referenzpunkt des zu optimierenden und herzustellenden Brillenglases. In einer bevorzugten Ausführungsform handelt es sich bei dem Referenzpunkt des Auges um einen Punkt, der sich bei Helligkeitsänderung relativ zur Netzhaut nicht verschiebt. Besonders bevorzugt werden dabei die Positionen des Apex des Auges direkt oder indirekt als Referenzpunkt ermittelt, d.h. es können die Apex-Position direkt gemessen werden, oder es werden Positionen eines anderen Punktes gemessen, dessen Position relativ zum Apex bekannt ist.

Vorzugsweise umfasst das Optimieren für jeden Referenzpunkt des Brillenglases ein Berechnen des Verlaufs eines Hauptstrahlen derart, dass er in einer (individuellen) Gebrauchsstellung des Brillenglases, welche vorzugsweise ebenfalls von Gebrauchsdaten festgelegt ist, durch die jeweilige Position des Referenzpunktes des Auges verläuft. Der Verlauf der Hauptstrahlen, also eine Hauptstrahliteration (Ray-Tracing) wie sie vorzugsweise auch in herkömmlichen Optimierungsverfahren zum Einsatz kommt, wird in dieser Ausführungsform also vorzugsweise unabhängig von der individuell, helligkeitsabhängig beeinflussten Pupillenposition durchgeführt. Die Berücksichtigung der individuellen Pupillenposition erfolgt dabei vorzugsweise durch eine Berechnung einer Wellenfront (Wave-Front-Tracing) in einer Umgebung des jeweiligen Hauptstrahls derart, dass die Position der Umgebung relativ zum Hauptstrahl gemäß dem bestimmten individuellen Einfluss der Helligkeit auf die Position der Pupille bestimmt und berücksichtigt wird. Insbesondere wird in dem der Berechnung zugrunde liegenden Modell des Strahlverlauf eine Aperturblende (Eintrittspupille des Auges) verwendet, deren Position relativ zum Hauptstrahl in Abhängigkeit von der Helligkeit eine individuelle Verschiebung aufweist. Die Wellenfront wird also in dieser Ausführungsform nicht notwendigerweise in einer um den Hauptstrahl zentrierten Umgebung, sondern in einer individuell verschobenen Umgebung ausgewertet.

Bei dieser bevorzugten Ausführungsform können die Positionen der Referenzpunkte bei verschiedenen Blickrichtungen insbesondere zusammen mit weiteren Rotationsparameter des Auges bestimmt werden, ohne dass die während dieser Messung vorliegende Helligkeit bekannt sein muss. Um dennoch den nötigen Bezug der Pupillenposition zum Referenzpunkt des Auges zu kennen, umfasst das Bestimmen eines individuellen Einflusses der Helligkeit des von dem zumindest einen Auge erfassten Lichts auf die Position der Pupille des zumindest einen Auges vorzugsweise ein Bestimmen der Position der Pupille relativ zum Referenzpunkt des Auges bei dem zumindest einen Helligkeitssollwert, vorzugsweise bei allen vorgegebenen Helligkeitssollwerten. Wie bereits oben ausgeführt, kann diese Bestimmung entweder durch eine direkte Messung beim jeweiligen Helligkeitssollwert oder beispielsweise durch eine Interpolation oder Extrapolation auf Basis von Modellen und/oder Messungen bei anderen Helligkeiten erfolgen.

In einer weiteren bevorzugten Ausführungsform wird die individuelle Position der Pupille für die Gebrauchssituation dadurch berücksichtigt, dass das Verfahren umfasst: Ermitteln, insbesondere Messen jeweils einer Position der Pupille des Auges für Blickrichtungen, welche den zumindest zwei Referenzpunkten des Brillenglases entsprechen, also für Blickrichtungen durch den jeweiligen Referenzpunkt des zu optimierenden und herzustellenden Brillenglases. Nachdem die Position der Pupille vorzugsweise von der Helligkeit abhängt und diese individuelle Abhängigkeit gerade berücksichtigt werden soll, wird die jeweilige Position der Pupille jeweils bei einer bestimmten oder bestimmbaren Helligkeit ermittelt. In einer bevorzugten Ausführungsform wird während dieses Vorgangs jeweils die Helligkeit gemäß dem jeweils vorgegebenen Helligkeitssollwert eingestellt. In einer anderen bevorzugten Ausführungsform wird die Helligkeit während der Messung lediglich ebenfalls gemessen. Aus einem insbesondere durch eine separate Messung bestimmten individuellen Einfluss der Helligkeit auf die Position der Pupille wird in diesem Fall eine Positionskorrektur der jeweiligen Pupillenposition für die verschiedenen Blickrichtung ausgehend von der gemessenen Pupillenposition durchgeführt.

Vorzugsweise umfasst das Optimieren in dieser bevorzugten Ausführungsform für jeden Referenzpunkt des Brillenglases ein Berechnen des Verlaufs eines Hauptstrahlen derart, dass er in einer (individuellen) Gebrauchsstellung des Brillenglases, welche vorzugsweise ebenfalls von Gebrauchsdaten festgelegt ist, durch die jeweilige gegebenenfalls korrigierte Position der Pupille des Auges verläuft. Falls also die Messung der Pupillenposition für die verschiedenen Blickrichtungen bei der Sollhelligkeit (Helligkeitssollwert) durchgeführt wurde, werden vorzugsweise die direkt gemessenen Positionswerte herangezogen. Falls andererseits Messungen bei einer anderen Helligkeit durchgeführt werden, werden die Pupillenposition zum Zweck der Hauptstrahliteration vorzugsweise entsprechend dem individuellen Einfluss der Helligkeit korrigiert.

Der Verlauf der Hauptstrahlen, also eine Hauptstrahliteration (Ray-Tracing) wie sie vorzugsweise auch in herkömmlichen Optimierungsverfahren zum Einsatz kommt, wird in dieser Ausführungsform also abhängig von der individuell, helligkeitsabhängig beeinflussten Pupillenposition durchgeführt. Auf Basis dieser Hauptstrahlberechnung erfolgt vorzugsweise eine Berechnung einer Wellenfront (Wave-Front-Tracing) in einer Umgebung des jeweiligen Hauptstrahls, deren Position vorzugsweise nicht von der Helligkeit abhängt. Die Größe dieser Umgebung hängt hingegen vorzugsweise durchaus in der oben beschriebenen Weise individuell von der Helligkeit ab. Insbesondere wird in dieser Ausführungsform in dem der Berechnung zugrunde liegenden Modell des Strahlverlauf eine Aperturblende (Eintrittspupille des Auges) verwendet, deren Position vorzugsweise zum Hauptstrahl zentriert ist.

Vorzugsweise umfasst das Bestimmen der erwarteten individuellen Position der Pupille und/oder das Bestimmen des individuellen Einflusses der Helligkeit auf die Position und/oder Größe der Pupille:

- Einstellen von Messkonditionen, bei denen die von dem zumindest einen Auge erfasste Helligkeit zumindest einem in der individuellen Gebrauchssituation festgelegten Helligkeitssollwert entspricht;
- Erfassen der Position bzw. Größe der Pupille des zumindest einen Auges (als den erwarteten individuellen Wert) unter den eingestellten Messkonditionen.

In einer alternativen, bevorzugten Ausführungsform umfasst das Bestimmen der erwarteten individuellen Position der Pupille und/oder das Bestimmen des individuellen Einflusses der Helligkeit auf die Position und/oder Größe der Pupille:

- Festlegen (Vorgeben) eines Zusammenhangs (z.B. als analytische Beschreibung in Form einer mathematischen Formel) zwischen der von dem zumindest einen Auge erfassten Helligkeit und der Position bzw. Größe der Pupille, wobei der festgelegte Zusammenhang zumindest einen individuellen (also individuell zu ermittelnden) Anpassparameter (auch freier Parameter oder Fitparameter bezeichnet) aufweist;
- Ermitteln (insbesondere Messen) einer Position und/oder Größe der Pupille zusammen mit einer von dem zumindest einen Auge erfassten Helligkeit. Es wird also insbesondere die während der Messung herrschende (mittlere) Helligkeit (oder diejenige Helligkeit, auf die sich die Pupille des zumindest einen Auges angepasst hat) als wesentlicher Bestandteil der Messkonditionen erfasst.
- Bestimmen des zumindest einen individuellen Anpassparameters aus der ermittelten Position und/oder Größe der Pupille sowie der zusammen damit ermittelten Helligkeit; und
- Ermitteln der bei dem vorgegebenen Helligkeitssollwert (bzw. den vorgegebenen Helligkeitssollwerten) erwarteten individuellen Position und/oder Größe der Pupille aus dem festgelegten Zusammenhang zwischen der von dem zumindest einen Auge erfassten Helligkeit und der Position bzw. Größe der Pupille unter Berücksichtigung des bestimmten individuellen Anpassparameters.

In einer bevorzugten Ausführungsform wird die zusammen mit einer Position und/oder Größe der Pupille ermittelte, von dem zumindest einen Auge erfasste Helligkeit mittels eines Helligkeitssensors (insbesondere direkt) gemessen.

In einer insbesondere dazu alternativen, bevorzugten Ausführungsform umfasst das Ermitteln einer Position und/oder Größe der Pupille zusammen mit einer von dem zumindest einen Auge erfassten Helligkeit:

- ein Anordnen eines Helligkeitsreferenzobjekts derart in der Nähe des zumindest einen Auges, dass das Helligkeitsreferenzobjekt derselben Helligkeit ausgesetzt ist wie das zumindest eine Auge;
- ein Erfassen von Bilddaten des zumindest einen Auges zusammen mit dem Helligkeitsreferenzobjekt (z.B. in einer einzigen Aufnahme oder - falls Blitzlicht verwendet wird - vorzugsweise unmittelbar hintereinander, wobei die Aufnahme des Helligkeitsreferenzobjekts ohne Blitzlicht erfolgt); und
- ein Bestimmen der Helligkeit aus der Darstellung des Helligkeitsreferenzobjekts in den Bilddaten.

Vorzugsweise umfasst wobei das Bestimmen der erwarteten individuellen Position der Pupille und/oder das Bestimmen des individuellen Einflusses der Helligkeit auf die Position und/oder Größe der Pupille ein Messen einer Position der Pupille relativ zu einem kopffesten Koordinatensystem. Vorzugsweise erfolgt das Messen mittels eines Videozentriersystems und unter Verwendung von extrinsischen Merkmalen (z.B. Brillenfassung, Markierungen an der Brillenfassung bzw. an einem Aufsteckelement). Je nachdem, ob diese Messung im Rahmen einer der bereits oben beschriebenen, bevorzugten Alternativen durchgeführt wird, wird vorzugsweise bereits die erwartete Position beim festgelegten Helligkeitssollwert gemessen, oder es wird eine Position bei einer beliebigen Helligkeit zusammen mit der dabei herrschenden Helligkeit gemessen, woraus anschließend der zumindest eine individuelle Anpassparameter für den vorgegebenen Zusammenhang bestimmt wird. Diese Vorgehensweise ist insbesondere dann bevorzugt, wenn die Messung der Position und/oder Größe der Pupille mittels eines Videozentriersystems erfolgt, welches darüber hinaus auch weitere individuelle Parameter (beispielsweise bezüglich der ausgewählten Brillenfassung) für die Brillenglasanpassung ermittelt.

Vorzugsweise umfasst das Bestimmen der erwarteten individuellen Position der Pupille und/oder das Bestimmen des individuellen Einflusses der Helligkeit auf die Position und/oder Größe der Pupille ein Messen einer Position der Pupille relativ zu einem ausgezeichneten Merkmal des zumindest einen Auges.

Vorzugsweise umfasst das Bestimmen der erwarteten individuellen Position der Pupille und/oder das Bestimmen des individuellen Einflusses der Helligkeit auf die Position und/oder Größe der Pupille ein Vorgeben einer Blickrichtung mittels eines Fixationsobjekt und/oder eines Fixationstargets.

In einer bevorzugten Ausführungsform wird eine Messung einer Position und/oder Größe der Pupille bei einer ersten Leuchtdichte im Bereich von etwa 3 cd/m$^2$ bis etwa 30 cd/m$^2$ und eine Messung einer Position und/oder Größe der Pupille bei einer zweiten (von der ersten verschiedenen) Leuchtdichte im Bereich von etwa 0,003 cd/m$^2$ bis etwa 30 cd/m$^2$, vorzugsweise im Bereich von etwa 0,003 cd/m$^2$ bis etwa 3 cd/m$^2$, besonders bevorzugt im Bereich von etwa 0,003 cd/m$^2$ bis etwa 0,3 cd/m$^2$, am meisten bevorzugt im Bereich von etwa 0,003 cd/m$^2$ bis etwa 0,03 cd/m$^2$, durchgeführt.

In einem weiteren Aspekt bietet die Erfindung eine Messvorrichtung zur Erfassung individueller Benutzerdaten, welche zumindest eine Position einer Pupille zumindest eines Auges festlegen, wobei die Messvorrichtung eine Beleuchtungseinrichtung umfasst, welche ausgelegt ist, eine von dem zumindest einen Auge erfasste Helligkeit zu bestimmen. Dies kann insbesondere auf zwei alternative, bevorzugte Weisen erfolgen. Entweder ist die Beleuchtungseinrichtung ausgelegt, die Helligkeit so zu steuern oder zu regeln dass eine vorgegebene Helligkeit erreicht wird, oder die Beleuchtungseinrichtung umfasst einen Sensor, der die von der (möglicherweise nicht steuerbaren) Beleuchtungseinrichtung und eventuellem Umgebungslicht bewirkte Helligkeit misst.

Außerdem umfasst die Messvorrichtung eine Bildaufnahmeeinrichtung, welche ausgelegt ist, Bilddaten von der Pupille zusammen mit einem Positionsreferenzpunkt, dessen Position relativ zum Auge, insbesondere zur Netzhaut, nicht von der Helligkeit abhängt, zu erfassen. Damit bietet die Erfindung eine Möglichkeit, Benutzerdaten zu erfassen, die einen individuellen Einfluss der Helligkeit auf die Position und/oder Größe der Pupille zumindest eines Auges umfassen. Diese Daten können dann genutzt werden, um eine verbesserte Anpassung einer Brille oder eines Brillenglases zu erreichen.

Vorzugsweise umfasst die Messvorrichtung einen Helligkeitssensor, der ausgelegt ist, die von dem zumindest einen Auge erfasste Helligkeit zu messen. Weiter bevorzugt umfasst die Messvorrichtung eine Gebrauchsdatenerfassungsschnittstelle zum Erfassen einer Vorgabe für zumindest einen Helligkeitssollwert und eine Beleuchtungssteuereinrichtung, welche ausgelegt ist, die Helligkeit der Beleuchtungseinrichtung derart zu steuern oder zu regeln, dass das von dem zumindest einen Auge erfasste Licht dem erfassten Helligkeitssollwert entspricht.

In einem weiteren Aspekt ist es nicht notwendig, dass die Messvorrichtung eine eigene Beleuchtungseinheit umfasst. Vielmehr kann die Umgebungshelligkeit genutzt werden und mittels eines Helligkeitssensors gemessen werden. In diesem Fall umfasst die Messvorrichtung somit:

- einen Helligkeitssensor, der ausgelegt ist, die von dem zumindest einen Auge erfasste Helligkeit zu messen; und
- eine Bildaufnahmeeinrichtung, welche ausgelegt ist, Bilddaten von der Pupille zusammen mit einem Positionsreferenzpunkt zu erfassen.

Vorzugsweise umfasst die Messvorrichtung ein Fixationstarget und/oder ein Fixationsobjekt und/oder eine Fixationsprojektionseinrichtung zur Steuerung der Blickrichtung des Auges, also insbesondere eine lichtemittierende Einrichtung zur Steuerung oder Lenkung der Blickrichtung zumindest eines Auges. Besonders bevorzugt wird das Fixationstarget und/oder das Fixationsobjekt und/oder die Fixationsprojektionseinrichtung von der Beleuchtungseinrichtung gebildet, wobei die vom Auge erfasste Helligkeit, welche die Position bzw. Größe der Pupille beeinflusst, zumindest überwiegend durch das Fixationstarget und/oder das Fixationsobjekt und/oder die Fixationsprojektionseinrichtung bereitgestellt wird. Besonders bevorzugt steuert bzw. regelt somit die Beleuchtungssteuereinrichtung die Helligkeit des Fixationstargets und/oder des Fixationsobjekts und/oder der Fixationsprojektionseinrichtung. Beim Erfassen der Position und/oder Größe der Pupille bzw. der Bilddaten der Pupille mittels der Messvorrichtung wird die relevante Helligkeit am Auge bzw. die Helligkeit des Fixationstargets und/oder des Fixationsobjekts und/oder der Fixationsprojektionseinrichtung bzw. die Helligkeit der Beleuchtungseinrichtung mit erfasst und insbesondere mit abgespeichert.

Vorzugsweise ist die Messvorrichtung als Videozentriersystem und/oder als Autorefraktometer und/oder Aberrometer und/oder Keratograph und/oder Tonograph und/oder Pachymeter ausgestaltet.

In einem weiteren Aspekt bietet die Erfindung ein Computerprogrammprodukt, insbesondere in Form eines Speichermediums oder einer Signalfolge, umfassend computerlesbare Anweisungen, welche, wenn geladen in einen Speicher eines Computers, vorzugsweise im Speicher einer Datenverarbeitungseinheit einer Vorrichtung gemäß der vorliegenden Erfindungen, insbesondere in einer ihrer hier beschriebenen bevorzugten Ausführungsformen, und ausgeführt von dem Computer (insbesondere der Vorrichtung), bewirken, dass der Computer (insbesondere die Vorrichtung) ein Verfahren gemäß der vorliegenden Erfindung, insbesondere in einer bevorzugten Ausführungsform durchführt.

Nach der Schilderung des Messprinzips gemäß eines Aspekts der Erfindung anhand bevorzugter Ausführungsformen werden nachfolgend verschiedene Aspekte bevorzugter Vorgehensweisen insbesondere im Hinblick auf Anwendungen dieser Messungen diskutiert. Schließlich wird auch beispielhaft dargelegt, wie die erfassten Daten vorzugsweise verwendet werden können, um die Zentrierung und insbesondere das Design der Brillengläser anzupassen, also insbesondere ein Brillenglas individuell zu optimieren.

In einem Aspekt bietet der Erfindung die Berücksichtigung der Position und/oder Größe der Pupille unter den Bedingungen, die in der späteren Anwendung tatsächlich vorliegen. Unter Bedingungen wird dabei zumindest auch die Helligkeit in der gewünschten Anwendungssituation verstanden. Darüber hinaus können aber auch andere Reize, die die Position bzw. Größe der Pupille beeinflussen können, eingesetzt werden. Dies betrifft beispielsweise die Akkommodation des Auges auf definierte Entfernungen (z.B. im unendlichen, im Nahbereich oder genebelt) sowie spezielle Fixations- oder Vergenzstellungen (z.B. monokulare oder binokulare Konvergenz durch Blicksteuerung über Fixationstargets oder Nahsehproben).

Die dazu erforderlichen Messungen werden vorzugsweise mit einer erfindungsgemäßen Vorrichtung durchgeführt, welche besonders bevorzugt als Videozentriersystem ausgelegt ist, also die Funktionalität eines Videozentriersystems aufweist, wobei vorzugsweise wenigstens eine der Messungen auch zur Bestimmung von Zentrierdaten oder weiterer individueller Parameter verwendet werden kann. Diese können durch Messungen mit anderen brillen- oder augenoptischen Geräten sowie dezidierten Geräten ergänzt bzw. ersetzt werden.

Für die notwendige Referenzierung verschiedener Messungen untereinander in Bezug auf die Pupillenmessungen, insbesondere in Bezug auf die Bestimmungen der Position der Pupille, können ausgezeichnete Merkmale des Auges, eine Fixierung der Blickrichtung oder referenzierende Messungen eingesetzt werden. Eine erfindungsgemäße Erfassung der Position bzw. Größe der Pupille für eine spezifizierte Bedingung kann durch die direkte Messung dieser Größen bei besagter Bedingung (z.B. der spezifizierten Helligkeit) oder durch die Berechnung aus Messungen bei davon unterschiedlichen Bedingungen (z.B. andere Helligkeiten) erfolgen.

Die Größe der Pupille wird dabei vorzugsweise gemäß einer der folgenden Arten definiert und entsprechend ermittelt:

- Fläche der tatsächlichen Pupille;
- Fläche des In- oder Umkreises der tatsächlichen Pupille;
- Fläche des den tatsächlichen Pupillenrand am besten beschreibenden Kreises;
- Gewichtete Fläche der tatsächlichen Pupille. Die Gewichtung erfolgt vorzugsweise als Funktion des Abstandes von einem ausgezeichneten Punkt zur Betonung spezieller Bereiche. Ein derart ausgezeichneter Punkt ist vorzugsweise die Pupillenmitte insbesondere gemäß einer der nachfolgenden Definitionen oder der Hornhautscheitel.

Anstelle eines Kreises können auch andere geometrische Formen (z.B. Ellipsen) herangezogen werden.

Unter der Position der Pupille wird dabei vorzugsweise ein ausgezeichneter Punkt der Pupille insbesondere gemäß einer der folgenden Definitionen verstanden:

- Schwerpunkt der tatsächlichen Pupillenfläche;
- Mittelpunkt des In- oder Umkreises der tatsächlichen Pupille;
- Mittelpunkt des den tatsächlichen Pupillenrand am besten beschreibenden Kreises;
- Schwerpunkt der gewichteten Fläche der tatsächlichen Pupille. Die Gewichtung erfolgt vorzugsweise als Funktion des Abstandes von einem ausgezeichneten Punkt zur Betonung spezieller Bereiche. Ein derart ausgezeichneter ist vorzugsweise die Pupillenmitte nach einer der obigen Definitionen (z.B. zur Betonung des zentralen Bereichs) oder der Hornhautscheitel.

Wie bereits erwähnt, werden in einer bevorzugten Ausführungsform wird bereits bei einer Messung einer individuellen Position und/oder Größe der Pupille mittels eines Videozentriersystems die Bedingungen (z.B. Lichtverhältnisse) geschaffen, die der späteren Gebrauchssituation entsprechen. Diese Messungen, insbesondere dabei erfasste Bilddaten, können auch zur Ermittlung der Zentrierdaten und individuellen Daten herangezogen werden.

Zusätzliche Aufnahmen (Bilddaten) können mit dem Videozentriersystem bei weiteren definierten Bedingungen (z.B. Lichtverhältnissen), die denen der späteren Anwendungen entsprechen, erzeugt und insbesondere nach Position und/oder Größe der Pupille ausgewertet werden, ohne eine komplette Auswertung im Sinn einer Videozentrierung zu durchlaufen.

In einer anderen bevorzugten Ausführungsform eines Erfassens von individuellen Benutzerdaten können auch *a priori* undefinierte Bedingungen (z.B. Lichtverhältnisse) eingesetzt werden, die falls für die jeweilige Anwendung erforderlich, gemessen werden können. Dazu können entweder ohnehin am Gerät vorhandene Beleuchtungseinrichtungen, wie beispielsweise Fixationstargets (also blickrichtungssteuernde Lichtfelder) oder Fixationsobjekte zur Vorgabe einer Blickrichtung, oder spezielle Beleuchtungseinrichtungen eines Videozentriersystems herangezogen bzw. vorgesehen werden. Selbstverständlich können auch externe Beleuchtungseinrichtungen, die auch Teil der Ladeneinrichtung beim Augenoptiker sein können, Verwendung finden. Analoges gilt für Fixationstargets, -objekte und -projektionen. Unter einer Fixationsprojektion wird dabei das in augenoptischen Geräten typischerweise eingesetzte abbildende System verstanden, dessen Abbildung der Betrachter fixiert bzw. auf dessen Abbildung er akkommodiert.

In der praktischen Anwendung eines Videozentriersystems bei Optiker ist es oft schwierig, die notwendigen Helligkeitsverhältnisse am Ort der Videozentrierung sicherzustellen, welche genau der tatsächlich für den Brillenträger erwarteten Gebrauchssituation entsprechen. So stehen Videozentriersysteme oft in einem Verkaufsraum, der nicht abgedunkelt werden kann. Deswegen nutzt eine weitere Ausführungsform eines Verfahrens zum Bestimmen einer bei dem zumindest einen Helligkeitssollwert auftretenden oder erwarteten individuellen Position der Pupille bei zumindest einer Blickrichtung des zumindest einen Auges mindestens ein weitere Messvorrichtung, mit der die Position der Pupille insbesondere in Bezug auf ein ausgezeichnetes Merkmal des Auges erfasst werden kann. Ein derartiges Gerät im Sinne einer bevorzugten Ausführungsform der Erfindung umfasst dabei mindestens eine Bildaufnahmeeinrichtung (Kamera), welche ausgelegt ist, zumindest Teile des Pupillenrandes zu erfassen. Außerdem umfasst die Messvorrichtung vorzugsweise eine (interne oder externe) Beleuchtungseinrichtung, mit der definierte Helligkeitsverhältnisse erzeugt werden können, d.h. die Messvorrichtung ist vorzugsweise ausgelegt, die Helligkeit der Beleuchtungseinrichtung zu steuern.

Vorzugsweise weist die Messvorrichtung auch ein Abschattelement zum Abschattung der Augenpartie auf, um auch in heller Umgebung für das Auge ein Umfeld mit geringer Helligkeit erzeugen zu können. Alternativ kann die Messvorrichtung auch in einem abgedunkeltem Raum (z.B. Refraktionsraum beim Optiker) oder einem abgeteilten Raumteil aufgestellt werden.

Zur Steuerung der Blickrichtung weist die Messvorrichtung vorzugsweise ein Fixationstarget und/oder ein Fixationsobjekt und/oder eine Fixationsprojektionseinrichtung auf, welche insbesondere ausgelegt ist, ein virtuelles Target zu erzeugen. In einer besonders bevorzugten Ausführungsform ist die Messvorrichtung gleichzeitig (also integral) als Autorefraktometer und/oder Aberrometer und/oder Keratograph und/oder Tonograph und/oder Pachymeter ausgestaltet.

Zur Zentrierung bzw. Optimierung von Brillengläsern ist die Kombination mit einer Videozentriermessung nicht unbedingt notwendig. Stattdessen können auch die Veränderungen der Position und/oder Größe der Pupille auch lediglich mit einer Messvorrichtung, wie sie die vorliegende Erfindung bietet, ermittelt werden, und die Einschleifposition des Glases kann unabhängig davon auf einem anderen Weg (z.B. manuelle Zentrierung nach Viktorix) bestimmt werden.

Unabhängig davon, ob die Bestimmung einer bei zumindest einem Helligkeitswert auftretende individuelle Position und/oder Größe der Pupille bzw. die Bestimmung eines individuellen Einflusses des Helligkeit auf die Position und/oder Größe der Pupille mittels eines insbesondere erfindungsgemäß ausgestatteten Videozentriersystems oder mittels einer anderen erfindungsgemäßen Messvorrichtung erfolgt, wird bei der Messung der Helligkeit am Ort des Probanden (Brillenträgers), insbesondere am Ort des Auges (also die vom Auge erfasst Helligkeit - auch als "relevante Helligkeit" bezeichnet), vorzugsweise gemäß einer der folgenden Möglichkeiten vorgegangen.

Gemäß einer bevorzugten Ausführungsform weist die Messvorrichtung (z.B. Videozentriersystem) einen insbesondere per Kabel oder kabellos angebunden Helligkeitssensor auf, der die relevante Helligkeit misst, oder die Helligkeit an einer Stelle misst, von der auf die relevante Helligkeit zurück gerechnet werden kann.

In einer anderen bevorzugten Ausführungsform wird die relevante Helligkeit mittels eines Helligkeitsreferenzobjekts ermittelt, welches von einer Bildaufnahmeeinrichtung zusammen mit dem zumindest einen Auge des Brillenträgers in Bilddaten erfasst wird. Aus der Helligkeit des Helligkeitsreferenzobjekts in den Bilddaten lässt sich damit auf die relevante Helligkeit schließen.

Um bei Verwendung eines Helligkeitsreferenzobjekts Fehlmessungen durch Spiegelungen oder ähnliche Effekte zu vermeiden wird vorzugsweise ein möglichst homogenes und diffus streuendes Objekt eingesetzt. Die räumliche Ausdehnung wird dabei vorzugsweise so gewählt, dass die Abbildung des Objekts wenigstens die Anzahl von Pixeln umfasst, die eine Bestimmung der Helligkeit mit der erforderlichen Genauigkeit erlaubt. Die spektrale Charakteristik wird dabei vorzugsweise so gewählt, dass unter Berücksichtigung der spektralen Charakteristik der Kamera bzw. der einzelnen Farbkanäle auf die relevante Helligkeit geschlossen werden kann.

Im einfachsten Fall kann ein derartiges Helligkeitsreferenzobjekt aus einem Stück weiter (oder farbiger) Pappe oder Plastik bestehen. Dieses kann auch aufklebbar gestaltet sein, um es so in einfacher und bequemer Weise an der Brillenfassung oder dem Probanden anbringen zu können.

Vorteilhaft lassen sich auch an der Brillenfassung oder eine Aufsteckbügel angeordnete extrinsische Positionsreferenzmarken derart gestalten, dass sie als Helligkeitsreferenzobjekte im Sinn dieser Erfindung verwendet werden können. Dadurch lassen sich auch - in besonders vorteilhafter Weise - Aufnahmen sowohl zur Ermittlung der Position des Augendrehpunkts als auch zur Bestimmung der Position und/oder Größe der Pupille unter Gebrauchsbedingungen verwenden und so mit wenigen Aufnahmen beide Größen bestimmen. In den meisten auf dem Markt befindlichen Videozentriersystemen kommen Aufsteckbügel zur geometrischen Kalibrierung von Kameraaufnahmen zum Einsatz. Auch diese können mit einem derartigen Helligkeitsreferenzobjekt versehen werden.

Weiterhin kann die ausgewählte Fassung als Helligkeitsreferenzobjekt verwendet werden, sofern ihre spektrale Charakteristik hinreichend genau bekannt ist oder im Vorfeld ermittelt wird. Das Helligkeitsreferenzobjekt kann auch ortsfest im Laden des Optikers an einer Position installiert sein, an der es in der Aufnahme erscheint und einen Rückschluss auf die relevante Helligkeit zulässt. Weitere mögliche Helligkeitsreferenzobjekte stellen die Merkmale der Person dar, die entweder bekannt sind oder nur einer geringen Schwankung zwischen verschiedenen Probanden unterliegen. Als Beispiel sei hier die Sklera genannt.

Aus dem Helligkeitswert der Pixel in der Aufnahme, die das Helligkeitsreferenzobjekt wiedergeben, wird unter Berücksichtigung der spektralen Charakteristik des Helligkeitsreferenzobjekts und der Kameraempfindlichkeit die relevante Helligkeit berechnet. In vielen Fällen werden die Messaufnahmen durch Blitzbeleuchtungen aufgehellt. Aufgrund der Zeitskalen hat eine derartige Zusatzbeleuchtung meist keinen Einfluss auf die Pupillengröße bzw. -position. Die aufgenommene Helligkeit des Helligkeitsreferenzobjekts wird aber von besagter Blitzbeleuchtung sehr wohl beeinflusst. In diesem Fall kann die Aufnahme des Helligkeitsreferenzobjekts zur Ermittlung der Helligkeit in einer vor- oder nachgelagerten Aufnahme ohne Blitzeinsatz, erfolgen, also unter der Helligkeit, auf die sich die Pupille in Größe und Position eingestellt hat.

Bei der Bestimmung der individuellen Positionen der Pupille für verschiedene Bedingungen (z.B. Helligkeit, Akkommodationszustand) ist es wichtig, die verschiedenen Positionen der Pupille relativ zueinander feststellen zu können. Um dies zu ermöglichen, werden im Folgenden verschiedene bevorzugte Ausführungsformen für Messverfahren vorgeschlagen, die auch miteinander kombiniert werden können. Dies ist insbesondere dann sinnvoll, wenn verschiedene Geräte eingesetzt werden.

In einer bevorzugten Ausführungsform erfolgt eine Referenzierung der Position der Pupille durch ein ausgezeichnetes Merkmal des Auges, welches insbesondere in Bezug auf eine Blickrichtung unabhängig von der Helligkeit ist. Vorzugsweise wird dabei als ausgezeichnetes Merkmal ein intrinsisches Merkmal wie Strukturen auf der Binde- oder der Hornhaut sowie dem Limbus oder ein extrinsisches wie ein Reflex einer definiert angebrachten Beleuchtungseinrichtung auf der Hornhaut oder Linse (z.B. "Purkinjereflex") herangezogen.

Wird bei dem Einsatz mehrerer Geräte (z.B. mindestens einer Messung mit einem Videozentriersystem und mindestens einer Messung mit einer anderen erfindungsgemäßen Messvorrichtung insbesondere in einer der beschriebenen bevorzugten Ausführungsformen) dasselbe ausgezeichnete Merkmal des Auges erfasst, können die relativ zu diesem unter den unterschiedlichen Bedingungen bestimmten Positionen der Pupille ohne zusätzlichen Abgleich direkt verwendet und miteinander verrechnet werden. Derartige Merkmale sind vorzugsweise die genannten intrinsischen Merkmale sowie Reflexe, die auf geometrisch äquivalenten Anordnungen der entsprechenden Beleuchtungseinrichtungen basieren.

In einer weiteren bevorzugten Ausführungsform erfolgt eine Referenzierung der Position der Pupille durch eine Fixieren der Blickrichtung. Wenn sich die Position des Kopfes zwischen einzelnen Messungen relativ zur Bildaufnahmeeinrichtung nicht ändert, können auch direkt die Positionen im Bildkoordinatensystem der einzelnen Aufnahmen relativ zueinander verwendet werden. Dies setzt jedoch voraus, dass die Blickrichtung fixiert bzw. gesteuert oder gemessen wird. Bei konstanter Blickrichtung können die relativen Daten direkt verwendet werden. Bei eventuellen Änderungen der Blickrichtungen zwischen den einzelnen Aufnahmen kann deren Effekt kompensiert werden.

Zur Steuerung der Blickrichtung werden vorzugsweise Fixationsobjekte oder Fixationstargets (d.h. mindestens in einer Ebene gerichtete Lichtfelder) herangezogen. Zur Messung der Blickrichtung können bekannte Methoden der Blickrichtungsermittlung (z.B. Messung der Purkinjereflexe) verwendet werden.

Sollte sich (beispielsweise durch Verwendung mehrerer Geräte) die Position des Kopfes relativ zur Bildaufnahmeeinrichtung zwischen einzelnen Messungen ändern, wird dies vorzugsweise kompensiert, indem die Positionen des Kopfes durch mindestens ein augenfremdes intrinsisches oder extrinsisches Merkmal festgestellt werden.

In einer weiteren bevorzugten Ausführungsform erfolgt die Referenzierung der Position der Pupille durch Messungen bei gleicher Helligkeit. Sollten beispielsweise bei der Messung mit einem Videozentriersystem und einer davon unabhängigen, anderen erfindungsgemäßen Messvorrichtung insbesondere gemäß einer bevorzugten Ausführungsform unterschiedliche ausgezeichnete Merkmale (z.B. verschiedene intrinsische Merkmale oder Reflexe mit unterschiedlicher Geometrie der Beleuchtungseinheiten) des Auges verwendet werden, kann die Referenzierung dadurch erfolgen, dass jeweils zumindest eine Aufnahmen mit beiden System bei - im Rahmen der notwendigen Genauigkeit - gleichen Bedingungen erfolgt. Dadurch kann die Position der jeweiligen ausgezeichneten Merkmale zueinander über die für diese gemeinsame Bedingung ermittelten Positionen der Pupille relativ zu der Position des entsprechenden ausgezeichneten Merkmals des Auges erfolgen. Diese gemeinsame Bedingung muss nicht notwendigerweise bekannt sein. Sie kann aber - z.B. um im Ablauf das Aufnehmen bei einer zusätzlichen Bedingung zu vermeiden - eine der später verwendeten Bedingungen sein.

In einer weiteren bevorzugten Ausführungsform wird aus der Pupillengröße auf die Position der Pupille geschlossen. Insbesondere wird zu zunächst vorzugsweise mittels einer erfindungsgemäßen Messvorrichtung ein individueller Zusammenhang zwischen der Pupillengröße und der Pupillenposition ermittelt. Vorzugsweise wird diesem Zusammenhang ein analytisches (z.B. lineares) Modell mit zumindest einem freien, an die individuelle Messung anpassbaren Parameter unterstellt. Vorzugsweise wird dieser ermittelte individuelle Zusammenhang dann auf weitere Messungen insbesondere mittels anderer Messsysteme (z.B. in einem Videozentriersystem) angewandt. So lässt sich dann beispielsweise aus Bilddaten, welche mit einem Videozentriersystem selbst bei einer vorab nicht bekannten Helligkeit ermittelt werden durch Auswertung der Pupillengröße auf die bei der Bildaufnahme vorgelegene Helligkeit und die für eine korrekte Zentrierung bei der in der Gebrauchssituation erwarteten Helligkeit erforderliche Korrektur der Pupillenposition rückschließen.

Im einfachsten Fall werden die Position und/oder Größe der Pupille unter den für Optimierung bzw. Zentrierung der Gläser gewünschten, also der späteren Gebrauchssituation entsprechenden Bedingungen gemessen. Es können aber auch Positionen und/oder Größen für einzelne Bedingungen ermittelt werden, für die keine direkte Messungen vorliegen. Vorzugsweise werden sogar kontinuierliche Verteilungen über zumindest Bereiche des Brillenglases ermittelt und verwendet. Damit wird vorzugsweise eine entfernungsabhängige Helligkeit mit einer sich daraus ergebenen entfernungsabhängigen Pupille in Gleitsichtgläsern analog zur Wirkungsverteilung zwischen Nah- und Fernpunkt berücksichtigt.

Vorzugsweise werden aus mindestens einer Messung der Position und/oder Größe der Pupille Parameter für ein (z.B. analytisch gegebenes) Modell gewonnen werden. Ein solches Modell beschreibt zusammen mit dem zumindest einen individuell ermittelten Parameter vorzugsweise einen individuellen Einfluss der Helligkeit des von dem zumindest einen Auge erfassen Lichts auf die Position und/oder Größe der Pupille. Bevorzugte Modelle sind beispielsweise lineare oder logarithmische Abhängigkeiten, Abhängigkeiten mit einem freien Parameter sowie Inter- bzw. Extrapolationen zwischen bzw. über mindestens zwei Stützwerten. Liegen mehr Messungen vor als das Modell an freien Parameter enthält, können zur Steigerung der Genauigkeit bzw. statistischen Sicherheit Anpassrechnungen (z.B. kleinste quadratische Abweichung) durchgeführt werden.

In einer bevorzugten Ausführungsform wird als Modell für die Größe der Pupille (Radius d in mm) in Abhängigkeit der Helligkeit (Leuchtdichte B in mL) verwendet:

$$\log (d) = a - b \quad (\log(B) + c)^3$$

Als Mittelwert für einen Großteil von Brillenträgern können dabei die Werte a = 0,8558, b = 0,000401 und c = 8,1 angesehen werden. Im Rahmen bevorzugter Ausführungsformen der Erfindung können nun durch mindestens eine Messung bei mindestens einer bekannten Helligkeit Werte für die Parameter a, b und/oder c angepasst oder ab drei Messungen bei bekannten Helligkeiten vorzugsweise sogar vollständig bestimmt werden.

Als ein bevorzugtes Beispiel für ein Modell der Position der Pupille wird ein linearer Zusammenhang zwischen der Position und der Größe der Pupille unterstellt, dessen Koeffizienten vorzugsweise individuell bestimmt werden. Vorzugsweise wird ein Koeffizient von etwa -0,07 bis etwa 0,14 Millimeter Verschiebung der Pupille pro Millimeter Dilatation (Größenänderung der Pupille) festgelegt bzw. ermittelt. Ein positiver Wert bedeutet dabei eine nasale Verschiebung bei Kontraktion. Dabei kann vorzugsweise das obige Modell für die Pupillengröße unterstellt werden.

Vorzugsweise werden auch weitere Reize (z.B. Akkommodation und Fokussierung, Blickrichtung und Vergenz, usw.) berücksichtigt, die die Position und/oder Größe der Pupille beeinflussen können. Die Reize können dabei entweder monokular oder binokular sowie in beliebigen Kombinationen gesetzt werden. So umfasst die festgelegte individuelle Gebrauchssituation primär zumindest einen Helligkeitssollwert und beschreibt damit die Lichtverhältnisse in der geplanten individuellen Anwendung der Brille. In einer bevorzugten Ausführungsform wird dabei eine Messung im Bereich der photopischen Helligkeit Leuchtdichten im Bereich von etwa 3 cd/m² bis etwa 30 cd/m²) und eine weitere im Bereich der mesopischen Helligkeit (Leuchtdichten im Bereich von etwa 0,003 cd/m² bis etwa 30 cd/m²) vorgenommen. Diese können durch Messungen an der Grenze zwischen den Bereichen sowie im skotopischen Bereich (Leuchtdichten im Bereich von etwa 3 · 10⁻⁶ cd/m² bis etwa 0,03 cd/m²) ergänzt werden.

Bei einer Berücksichtigung von Akkommodation und Fokussierung können beispielsweise reale Objekte (z.B. Fixationsobjekte oder Nahleseproben) oder Abbildungen (z.B. die in augenoptischen Geräten eingesetzten Projektionssysteme auf Basis eines Dias oder eines CCDs) eingesetzt werden. Damit lässt sich die Akkommodation des Auges auf vorgegebene Entfernungen (z.B. im Unendlichen, im Nahbereich oder "genebelt" also nicht akkommodierbar) steuern.

Weiterhin können zur Steuerung der Blickrichtung bzw. zum Erzeugen spezieller Fixations- oder Vergenzstellungen reale Objekte (z.B. Fixationsobjekte, Nahleseproben), Abbildungen oder Fixationstargets (d.h. mindestens in einer Ebene Polarisierte Lichtfelder) eingesetzt werden. Dabei kann auch die Verrollung berücksichtigt werden.

Die so gewonnenen Informationen über Position und/oder Größe der Pupille werden nun insbesondere zur Zentrierung und/oder Optimierung der Brillengläser herangezogen. Im Folgenden werden einige Möglichkeiten ausgeführt, die auch kombiniert werden können.

So erfolgt vorzugsweise eine Berücksichtigung der tatsächlichen Position bei der Bestimmung der geometrischen Lage der Durchblickspunkte. Im einfachsten Fall werden dabei die gewonnen Informationen über die Position und/oder Größe der Pupille zur Bestimmung der Position einzelner Bezugs- bzw. Durchblickspunkte (wie dem Nah- oder Fernbezugspunkt) herangezogen. Dies kann auch durch die Korrektur anderweitig ermittelter Größen wie Einschleifhöhe und monokulare Pupillendistanz erfolgen. In einer bevorzugten Ausführungsform werden dem Nah- bzw. Fernbezugspunkt bestimmte Helligkeiten und/oder Akkommodationszustände zugeordnet.

Insbesondere in Verbindung mit einem Modell zur Beschreibung des Einflusses der Helligkeit auf die Position und/oder Größe der Pupille kann auch der genaue Verlauf (horizontal und vertikal) der Hauptblicklinie in Gleitsichtgläsern kontinuierlich in jedem Punkt gemäß den lokal vorgesehenen Bedingungen (z.B. Akkommodationszustand bzw. Helligkeit) vollständig optimiert werden. Besonders bevorzugt erfolgt bei der Optimierung eines Brillenglases eine Berücksichtigung der tatsächlichen Position und/oder Größe der Pupille unter den vorgesehenen Bedingungen (z.B. Akkommodationszustand bzw. Helligkeit).

Wie bereits weiter oben erwähnt, bietet die Erfindung in einem weiteren Aspekt die Möglichkeit, bei der Optimierung und Herstellung von Brillengläsern das Pupillenspiel (also insbesondere die vorzugsweise individuelle Abhängigkeit der Größe und/oder Position der Pupille von der individuellen Gebrauchssituation) und die damit verbundene Änderung der Refraktion (bzw. die davon abhängige Refraktion) des Auges zu berücksichtigen, ohne dass die Abbildungsfehler höherer Ordnung (HOA) des individuellen Auges bekannt sein bzw. individuell gemessen werden müssen. Herkömmlich werden letztere in der Regel mit einem aufwendigen Wellenfrontmessgerät ermittelt, welches oftmals nicht zur Verfügung steht.

Die Entwicklung von individuell optimierten Brillengläsern, insbesondere von Gleitsichtgläsern, erlaubt es mittlerweile, auf die individuellen Merkmale des Brillenträgers wie Pupillendistanz, Hornhautscheitelabstand, und individueller Gebrauchsstellung der Brille zu berücksichtigen. Die neuste Brillenglas-Generation ist sogar in der Lage, bei der Optimierung der Gläser zusätzlich Aberrationen höherer Ordnung, sog. "higher order aberrations" (HOA) zu berücksichtigen, die bei der Brechung des Lichts an den Grenzflächen des Brillenglases und Auges entstehen.

Die Optimierung solcher Brillengläser beinhaltet insbesondere in einer bevorzugten Ausführungsform der vorliegenden Erfindung die Propagation und Brechung einer individuell für jedes Auge bestimmten Wellenfront, die sich über eine vorzugsweise individuell bestimmt Pupille erstreckt und sowohl aus Aberrationen höherer Ordnung (HOA) als auch Aberrationen niedriger Ordnung (LOA) besteht. Sowohl die LOA als auch die HOA des Brillenglases werden durch die Gebrauchsstellung des Brillenglases beeinflusst und gehen in die Optimierung ein, ebenso gehen die HOA und die LOA des Auges sowie die subjektiv ermittelte Refraktion in die Optimierung ein.

Da die Wellenfrontdurchrechnung untrennbar mit der Pupillengröße verbunden ist, geht auch diese in die Optimierung des Brillenglases mit ein. Dabei kann - bedingt durch physiologische Phänomene wie z.B. Lichtreaktion oder Pupillennahreaktion - die in der Gebrauchssituation vorliegende Pupillengröße eine Funktion einer oder mehrerer Größen wie z.B. der Blickrichtung, der Konvergenz, der Helligkeit der betrachteten Szene und/oder des Objektabstands sein. Vor allem in dieser Hinsicht wird durch eine erfindungsgemäße, individuelle Berücksichtigung von Größe und/oder Position der Pupille eine Verbesserung der Anpassung eines Brillenglases erreicht. Um nicht nur die Größe und/oder Position der Pupille, sondern auch die Wellenfront individuell zu bestimmen, könnte beispielsweise ein Aberrometer verwendet werden. Solche Geräte sind zwar für den Augenoptiker in der Regel teuer in der Anschaffung und deshalb nicht weit verbreitet, bieten aber eine sehr präzise Möglichkeit zur Bestimmung der individuellen Wellenfrontaberration des Auges.

Wie bereits erwähnt, bietet die Erfindung in einem Aspekt einen alternativen Ansatz, der es ermöglicht, die Vorteile der neusten Brillenglastechnologie flächendeckend anbieten zu können, ohne dass überall ein Aberrometer verfügbar sein muss. Wie bereits erwähnt, müssen dabei die Abbildungsfehler höherer Ordnung (HOA) des individuellen Auges nicht bekannt sein bzw. nicht individuell gemessen werden. Dies wird insbesondere erreicht, indem auf eine individuelle Wellenfrontmessung verzichtet wird, und die zur Optimierung der Gläser benötigte individuell ermittelte Wellenfront und unter Umständen auch die individuell ermittelte Pupillengröße durch Modellannahmen ersetzt werden. Durch die in dem hier beschriebenen Verfahren verwendeten Modellannahmen erreicht man gleichzeitig, dass sich im Vergleich zu einem konventionellen Brillenglas die Wahrscheinlichkeit erhöht, dass eine Einzelperson eine gute Versorgung mit einem Brillenglas erhält. Dadurch verbessert sich durch ein Verfahren gemäß dieses Aspektes der Erfindung insgesamt die Versorgung einer Gruppe von Personen mit Brillengläsern.

Zur detaillierteren Beschreibung dieses Aspektes der Erfindung werden nachfolgend einige Begriffe verwendet, die insbesondere wie folgt verstanden werden sollen:

Mit dem Begriff "Daten" (Daten einer Gruppe von Augen bzw. eines einzelnen Auges) sind vorzugsweise mindestens eine Art von folgenden Daten gemeint: Wellenfrontdaten, Helligkeit, Pupillendaten, Refraktionsdaten, "sonstige Daten". Die einzelnen Arten von Daten werden im Folgenden erklärt.

So beschreiben die "Wellenfrontdaten" die insbesondere bei einer Wellenfrontmessung (oder in erfindungsgemäßer Weise auf Basis einer Modellannahme) bestimmte Wellenfront. Sie liegen vorzugsweise als Koeffizienten der Zernikepolynome, sog. Zernike-Koeffizienten, bei einer definierten Pupillengröße vor und beinhalten Aberrationen niedriger Ordnung (LOA) wie insbesondere Prisma, Sphäre, Astigmatismus, sowie Aberrationen höherer Ordnung (HOA) wie insbesondere Dreiblatt, Koma und/oder sphärische Aberration. Die Zernike-Koeffizienten können für eine Standard-Pupillengröße angegeben sein, die sich von der tatsächlich (individuell) bestimmten bzw. gemessenen Pupillengröße unterscheiden kann.

Mit "Helligkeit" wird insbesondere die Beleuchtungsstärke des auf das Auge auftreffenden bzw. des vom Auge erfassten Lichts bezeichnet.

"Pupillendaten" charakterisieren die Eintrittspupille des Auges und umfassen insbesondere den Pupillenparameter Pupillengröße (z.B. Pupillenradius oder Pupillendurchmesser) und bevorzugt auch einen oder mehrere der folgenden Daten: Pupillenmitte (z.B. Lage des Schwerpunkts der Pupillenfläche relativ zum Apex), Amplitude des Hippus, sowie Änderung der Pupillengröße bei sich ändernder Helligkeit der Umgebung. Die Pupillengröße und bevorzugt auch die Pupillenmitte müssen bei der Wellenfrontmessung vorliegen, diese und andere Pupillendaten können aber auch mit anderen Messgeräten bestimmt werden. Insbesondere kann sich die in den Pupillendaten vorhandene Pupillengröße von der Standard-Pupillengröße unterscheiden, für welche die Zernike-Koeffizienten der Wellenfrontdaten angegeben sind.

Die "Refraktionsdaten" beinhalten vorzugsweise Sphäre, Zylinder, Achse für die Sehentfernung unendlich oder für eine andere Sehentfernung, bei der die Refraktion durchgeführt worden ist, sowie die Addition, bzw. Sphäre, Zylinder, Achse, welche bei einer endlichen Leseentfernung bestimmt werden (z.B. 40 cm). Sphäre, Zylinder und Achse könne dabei auch in einer äquivalenten Darstellung als sog. Powervektoren vorliegen.

Unter den oben genannten "sonstigen Daten" des Auges werden im Folgenden mindestens eine Art folgender Daten verstanden: bei der Refraktion (bzw. Refraktionsbestimmung) auf das Auge treffende (bzw. vom Auge erfasste) Helligkeit, bei der Messung von Pupillendaten auf das Auge treffende (bzw. vom Auge erfasste) Helligkeit, Akkommodationszustand des Auges, sowie Alter des Auges (also des Brillenträgers).

Somit bietet die Erfindung in einem Aspekt ein Verfahren zum Optimieren und Herstellen eines Brillenglases für zumindest ein Auge eines Brillenträgers, vorzugsweise gemäß einem der oben beschriebenen Aspekte. Dabei umfasst das Verfahren ein Bereitstellen einer Verteilung von Daten, insbesondere von gemessenen Daten, einer Vielzahl von Augen, welche insbesondere das zumindest eine Auge des Brillenträgers nicht enthalten. Diese Daten der Vielzahl von Augen bilden damit insbesondere zumindest einen Teil eines (statistischen) Datensatzes, welcher als Grundlage für ein statistisches Modell von Zusammenhängen zwischen unterschiedlichen physikalischen und/oder physiologischen Parametern beschreibt. Dazu unterscheiden sich die Daten in der Verteilung von Daten bzw. die Augen der Vielzahl von Augen zumindest teilweise in physikalischen und/oder physiologischen Parametern. Die Verteilung von Daten kann als eine Stichprobe oder in Form von analytischen Modellen vorliegen.

In einer bevorzugten Ausführungsform umfasst das Bereitstellen einer Verteilung von Daten ein Bereitstellen einer Verteilung von Abbildungsfehlern höherer Ordnung (HOA) einer Vielzahl von Augen, die vorzugsweise von den Parametern Pupillendaten und/oder Refraktionsdaten und/oder sonstigen Daten abhängt.

Außerdem umfasst das Verfahren in diesem Aspekt der Erfindung ein Bereitstellen (insbesondere Messen) bzw. Bestimmen von physikalischen und/oder physiologischen Daten für das zumindest eine Auge des Brillenträgers. Vorzugsweise umfasst das Bereitstellen bzw. Bestimmen von physikalischen und/oder physiologischen Daten für das zumindest eine Auge des Brillenträgers ein Bereitstellen (insbesondere Messen) bzw. Bestimmen von Refraktionsdaten des zumindest einen Auges insbesondere für zumindest zwei verschiedene Referenzpunkte, vorzugsweise gemäß zumindest einiger oben beschriebener Details der weiteren Aspekte der vorliegenden Erfindung. Dabei können diese bereitgestellten (insbesondere gemessenen) bzw. bestimmten physikalischen und/oder physiologischen Daten insbesondere direkt solche Parameter betreffen bzw. umfassen, von denen die bereitgestellte Verteilung der Daten einer Vielzahl von Augen direkt abhängt oder diese Parameter werden aus den bereitgestellten (insbesondere gemessenen) bzw. bestimmten physikalischen und/oder physiologischen Daten anhand weiterer funktionaler Zusammenhänge ermittelt, wie dies anhand bevorzugter Ausführungsformen weiter unten noch genauer beschrieben wird.

Außerdem umfasst das Verfahren gemäß diesem Aspekt der Erfindung ein Ermitteln wahrscheinlichster Daten des zumindest einen Auges bei mindestens einer physikalischen und/oder physiologischen Bedingung. Insbesondere werden dabei aus den bereitgestellten (insbesondere gemessenen) bzw. bestimmten physikalischen und/oder physiologischen Daten (Bedingungen) für das zumindest eine Auge des Brillenträgers und/oder aus weiteren physikalischen und/oder physiologischen Bedingungen (insbesondere eine individuelle Gebrauchssituation betreffend) auf die gemäß der bereitgestellten (statistischen) Verteilung der Daten für das zumindest eine Auge wahrscheinlichsten Werte dieser Daten rückgeschlossen. In einer bevorzugten Ausführungsform wird dabei auf die wahrscheinlichsten Wellenfrontdaten des zumindest einen Auges des Brillenträgers bei mindestens einem Paar aus Pupillendaten und Akkommodationszustand rückgeschlossen. Die derart ermittelten wahrscheinlichsten Daten können dann direkt oder indirekt bei der Berechnung oder Optimierung des Brillenglases verwendet werden.

Auch wenn sich für diesen Aspekt der Erfindung, wonach anstelle einer individuellen Messung von Daten auf ein (statistisches) Modell (bzw. auf statistische Modelle) zurückgegriffen wird, vor allem bei der Bestimmung von Wellenfrontdaten die oben diskutierten Verbesserungen und Vereinfachungen ergeben, ist dieser Aspekt der Erfindung nicht auf die Nutzung im Zusammenhang mit Wellenfrontdaten beschränkt. Auch andere (statistische) Zusammenhänge zwischen physikalischen und/oder physiologischen Daten bzw. Parametern können auf diese Weise zur Bestimmung entsprechender individueller Daten herangezogen werden. Dies betrifft insbesondere ein Bestimmen einer erwarteten individuellen Position der Pupille und/oder ein Bestimmen eines individuellen Einflusses der Helligkeit des von dem

14

zumindest einen Auge erfassten Lichts auf die Position der Pupille des zumindest einen Auges und/oder ein Bestimmen einer jeweils bei den festgelegten Helligkeitssollwerten auftretenden oder erwarteten individuellen Größe der Pupille. Dabei werden die zu bestimmenden Daten auf Basis des statistischen Datensatzes aus individuell ermittelten Parametern für das Auge des Brillenträgers bestimmt.

Weitere Details insbesondere zu bevorzugten Ausführungsformen der Erfindung werden nachfolgend mit Bezug auf begleitende Zeichnungen beispielhaft beschrieben. Dabei zeigen:

Fig. 1    eine schematische Darstellung des Ablaufes eines Verfahrens gemäß einer bevorzugten Ausführungsform der vorliegenden Erfindung;

Fig. 2    eine schematische Darstellung des Ablaufes eines Verfahrens gemäß einer weiteren bevorzugten Ausführungsform der vorliegenden Erfindung; und

Fig. 3    eine schematische Darstellung weiterer Details des Ablaufes eines Verfahrens gemäß einer weiteren bevorzugten Ausführungsform der vorliegenden Erfindung, insbesondere gemäß Fig. 2.

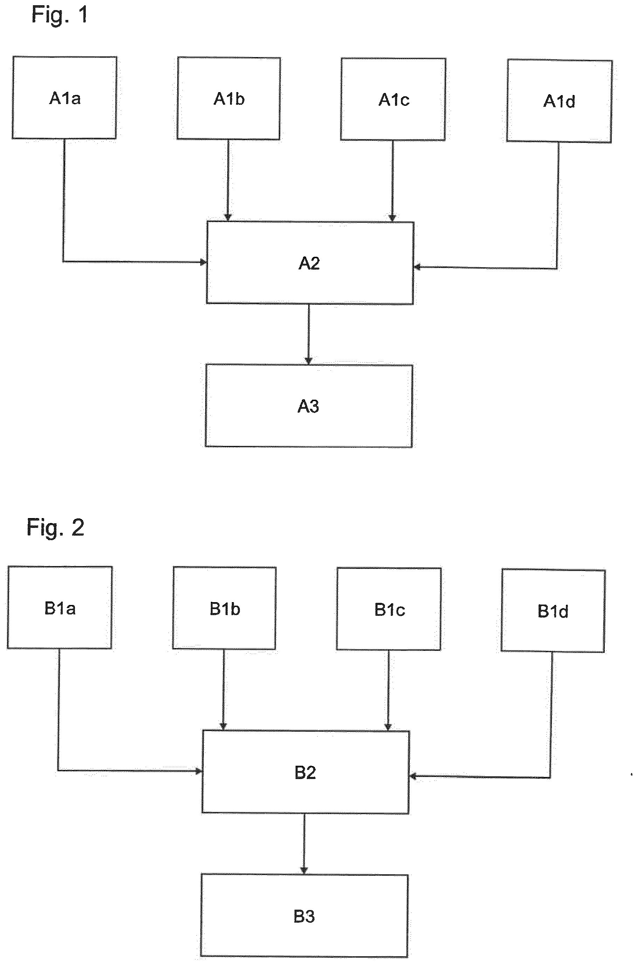

In einer bevorzugten Ausführungsform umfasst ein Verfahren (insbesondere ein Verfahren zum Anpassen eines individuellen Brillenglases bzw. ein Verfahren zum Optimieren und Herstellen eines Brillenglases) insbesondere die in Fig. 1 dargestellten Verfahrensschritte, die im Folgenden erläutert werden:

Schritt A 1 a: Bereitstellen einer Verteilung der Daten einer Gruppe (Vielzahl) von Augen, die von physikalischen und/oder physiologischen Parametern abhängt. Die Verteilung der Daten einer Gruppe von Augen kann als eine Stichprobe oder in Form von analytischen Modellen bereitgestellt werden. Sie stellt die für eine Gruppe von Augen bekannte statistische Information dar. Diese bereitgestellte Verteilung von Daten wird daher hier auch als Statistikdatensatz bezeichnet. Mögliche Daten einer Gruppe von Augen sind z.B. Wellenfrontdaten, Helligkeit, Pupillendaten, Refraktionsdaten oder sonstige Daten von Augen, sowie deren Kombinationen. Vorzugsweise sind in der Verteilung ebenfalls die statistischen Zusammenhänge zwischen mindestens einem Bestandteil dieser Daten und mindestens einer weiteren Einflussgröße, im Folgenden Parameter genannt, dargestellt. Die Parameter können physikalischer oder physiologischer Natur sein. Mögliche Parameter sind Pupillendaten, Refraktionsdaten, Helligkeiten, sonstige Daten oder Kombinationen hiervon.

Ein mögliches analytisches Modell einer solchen Verteilung gemäß einer bevorzugten Ausführungsform besteht aus einer vektorwertigen Regressionsfunktion und einem multivariat normalverteilten Regressionsfehler. Sowohl die Regressionsfunktion als auch die Kovarianzmatrix des Regressionsfehlers ist dabei eine vektor- bzw. matrixwertige Funktion mindestens eines der Parameter. Die Regressionsfunktion und Kovarianzmatrix erhält man typischerweise durch minimieren der mit der Kovarianzmatrix gewichteten Fehlerquadrate einer Stichprobe.

Jede Komponente der Regressionsfunktion kann in der Form eines abschnittsweise definierten Polynoms der Parameter vorliegen, und gibt die im Mittel vorhandene Abhängigkeit des dieser Komponente zugeordneten Typs der Daten von den Parametern, an. Insbesondere kann eine solche Funktion abschnittsweise eine konstante Funktion, eine lineare Funktion oder eine quadratische Funktion der Parameter sein. Der multivariat normalverteilte Regressionsfehler kann, muss aber nicht, eine nichtdiagonale Kovarianzmatrix besitzen, die ebenfalls eine Funktion der Parameter sein kann. Eine solche Funktion (d.h. jedes Element der Kovarianzmatrix) kann wie die Regressionsfunktion selbst als abschnittsweise konstante Funktion, lineare Funktion oder eine quadratische Funktion der Parameter genähert sein. Hängt insbesondere Regressionsfehler nicht von den Parametern ab, so ist die Kovarianzmatrix eine konstante Funktion der Parameter. Ergeben sich keine statistischen Zusammenhänge zwischen einzelnen Komponenten, so ist die Kovarianzmatrix diagonal.

Schritt A1b: Bereitstellen von physikalischen und/oder physiologischen Daten eines einzelnen Auges. In diesem Schritt werden physikalische oder physiologische Daten eines einzelnen Auges bereitgestellt, für das ein Brillenglas berechnet werden soll.

Schritt A1c: Gegebenenfalls Bereitstellen wahrscheinlichster Daten WD1 des einen Auges, die einem oder mehreren Parametern der Verteilung aus Schritt A1a entsprechen. Wenn nötig, werden die wahrscheinlichsten Daten WD1 des Auges, für die das eine Brillenglas berechnet werden soll, bereitgestellt. Diese wahrscheinlichsten Daten des Auges entsprechen einem oder mehreren Parametern, von denen die in Schritt A1a bereitgestellte Verteilung abhängt.

Schritt A1d: Gegebenenfalls Bereitstellen mindestens eines funktionalen Zusammenhangs zwischen den Parametern der in Schritt A1a bereitgestellten Verteilung und den in Schritt A1b und/oder in Schritt A1c bereitgestellten Daten des einen Auges. Sofern im nachfolgenden Schritt A2 ein statistischer Zusammenhang zwischen einem oder mehreren Parametern der in Schritt A1a bereitgestellten Verteilung und einem oder mehreren in Schritt A1b bereitgestellten physikalischen und/oder physiologischen Daten, oder den in Schritt A1c bereitgestellten wahrscheinlichsten Daten benötigt wird, es aber nicht möglich ist, einen direkten statistischen Zusammenhang bereitzustellen, so muss zusätzlich ein funktionaler Zusammenhang bereitgestellt werden. Der funktionale Zusammenhang kann dabei aus zusätzlichen Quellen gewonnen werden und stellt einen im Mittel vorhandenen statistischen Zusammenhang dar.

Schritt A2: Rückschließen auf die wahrscheinlichsten Daten WD2 des einen Auges bei mindestens einer physikalischen und/oder physiologischen Bedingung. Falls benötigt, werden zunächst (ein oder mehrere) funktionale Zusammenhänge aus Schritt A1d benutzt, um Daten und/oder wahrscheinlichste Daten WD1, die für das Auge in Schritt A1b bzw. A1c bereitgestellt wurden, in die Parameter umzurechnen, von denen die in Schritt A1a bereitgestellte Verteilung abhängt.

Als nächstes wird vorzugsweise eine bedingte Verteilung der Daten einer Gruppe von Augen berechnet, indem die in Schritt A1a bereitgestellte Verteilung der Daten an den in Schritt A1b bereitgestellten Daten des Auges und/oder den in Schritt A1c bereitgestellten wahrscheinlichsten Daten WD1 dieses Auges ausgewertet wird, die gegebenenfalls vorher im Hilfe der in Schritt A1d bereitgestellten funktionalen Zusammenhänge berechnet wurden.

Ist die Verteilung der Daten einer Gruppe von Augen als Stichprobe gegeben, so ist die bedingte Verteilung derjenige Teil der Stichprobe, dessen Parameter sich hinreichend wenig von den in den Schritten A1b und/oder A1c bereitgestellten Daten bzw. wahrscheinlichsten Daten WD1 unterscheiden. Ist die Verteilung der Daten einer Gruppe von Augen als analytische Approximation vorhanden, so berechnet sich die bedingte Verteilung durch Einsetzen der wahrscheinlichsten Daten WD1 und/oder der physikalischen/physiologischen Daten des einzelnen Auges in die analytische Approximation. Ist insbesondere die Verteilung der Daten der Gruppe von Augen als Regressionsmodell gegeben, so ergibt sich die bedingte Verteilung der Daten durch Einsetzen der Daten bzw. wahrscheinlichsten Daten WD1 des besagten Auges in die Regressionsfunktion sowie in die Kovarianzmatrix.

Vorzugsweise ergeben sich in analoger Weise die bedingten Verteilungen der in Schritt A1a bereitgestellten Daten bei einer oder mehreren physikalischen und/oder physiologischen Bedingungen, welche bei der Berechnung des Brillenglases benötigt werden. Dazu wird die vorhin ermittelte bedingte Verteilung bei den jeweiligen physikalischen und/oder physiologischen Bedingungen ausgewertet. Die wahrscheinlichsten Daten WD2* des einzelnen Auges bei diesen Bedingungen sind durch die Maxima dieser bedingten Verteilung gegeben. Die oben berechneten wahrscheinlichsten Daten WD2* können unter Berücksichtigung der in den Schritten A1b und A1c bereitgestellten Daten zu den wahrscheinlichsten Daten WD2 des einzelnen Auges ergänzt werden.

Schritt A3: Direktes oder indirektes Verwenden der wahrscheinlichsten Daten WD2 des einen Auges aus Schritt A2 bei der Berechnung oder Optimierung des Brillenglases. Die im vorigen Schritt ermittelten wahrscheinlichsten Daten WD2 des einen Auges werden nun bei der Berechnung eines Brillenglases verwendet. Dies kann entweder direkt geschehen, sodass der Schritt A3 die Berechnung des Brillenglases beinhaltet. In die Berechnung fließt außer den wahrscheinlichsten Daten WD2 auch andere Information ein, wie z.B. die individuell ermittelte Refraktion des Auges. Die wahrscheinlichsten Daten WD2 können aber auch indirekt in die Berechnung des Brillenglases einfließen, indem sie in einem weiteren Verfahrensschritt ähnlich dem Schritt A1c verwendet werden.

Eine weitere bevorzugte Ausführungsform eines Verfahrens, welches insbesondere als bevorzugter Spezialfalls des mit Bezug auf Fig. 1 beschriebenen Verfahrens angesehen werden kann, wird nachfolgend mit Bezug auf Fig. 2 beschrieben. Das hier beschriebene Verfahren umfasst insbesondere die in Fig. 2 dargestellten Verfahrensschritte.

Schritt B1a: Bereistellen einer Verteilung der HOA einer Gruppe von Augen, die vorzugsweise von den Parametern Pupillendaten und/oder Refraktionsdaten und/oder sonstigen Daten abhängt. Die Verteilung kann als eine Stichprobe oder in Form von analytischen Modellen vorliegen. Sie stellt die für eine Gruppe von Augen bekannte statistische Information über die für eine Standard-Pupille angegebene Zernike-Koeffizienten der Aberrationen höherer Ordnung (HOA) dar. Wenn möglich sollen in der Verteilung ebenfalls die statistischen Zusammenhänge zwischen mindestens einem für eine Standard-Pupille angegebenen Zernike-Koeffizienten und mindestens einer weiteren Einflussgröße, im folgenden Parameter genannt, dargestellt sein. Mögliche Parameter sind Pupillendaten, Refraktionsdaten und/oder sonstige Daten.

Ein mögliches analytisches Modell einer solchen Verteilung besteht aus einer vektorwertigen Regressionsfunktion und einem multivariat normalverteilten Regressionsfehler. Sowohl die Regressionsfunktion als auch die Kovarianzmatrix des Regressionsfehlers ist dabei eine vektor- bzw. matrixwertige Funktion mindestens eines der folgenden Parameter: Pupillenparameter, der Refraktionsdaten in Powervektor-Darstellung, der Addition, dem Alter, dem Akkommodationszustand, den LOA der auf eine Standard-Pupille skalierten Zernike-Koeffizienten. Die Refraktionsdaten können dabei subjektiv bestimmte Refraktionsdaten sein, oder aber objektive Refraktionsdaten, die in geeigneter Weise ais den Zernike-Koeffizienten und Pupillengrößen bestimmt wurden. Die Regressionsfunktion und Kovarianzmatrix erhält man typischerweise durch Minimieren der mit der Kovarianzmatrix gewichteten Fehlerquadrate einer Stichprobe.

Jede Komponente der Regressionsfunktion kann in der Form eines abschnittsweise definierten Polynoms der oben genannten Parameter vorliegen, und gibt die im Mittel vorhandene Abhängigkeit des dieser Komponente zugeordneten Zernike-Koeffizienten von den Parametern an, wobei der Zernike-Koeffizient für eine Standard-Pupille angegeben ist. Insbesondere kann eine solche Funktion abschnittsweise eine konstante Funktion, eine lineare Funktion oder eine quadratische Funktion der Parameter sein. Der multivariat normalverteilte Regressionsfehler kann, muss aber nicht, eine nichtdiagonale Kovarianzmatrix besitzen, die ebenfalls eine Funktion der oben genannten Parameter sein kann. Eine solche Funktion (d.h. jedes Element der Kovarianzmatrix) kann wie die Regressionsfunktion selbst als abschnittsweise konstante Funktion, lineare Funktion oder eine quadratische Funktion der Parameter genähert sein. Hängt insbesondere der Regressionsfehler nicht von den Parametern ab, so ist die Kovarianzmatrix eine konstante Funktion der Parameter. Ergeben sich keine statistischen Zusammenhänge zwischen einzelnen Zernike-Koeffizienten, so ist die Kovarianzmatrix diagonal.

Ein bevorzugter Spezialfall eines solchen analytischen Modells ist die Verteilung der Zernike-Koeffizienten $C_3^{-3}$ bis $C_5^{5}$, wobei die Komponenten der Regressionsfunktion für alle Koeffizienten bis auf $C_4^{0}$ konstant sind, und die der sphärischen Aberration $C_4^{0}$ entsprechende Komponente eine stückweise lineare Funktion des Alters ist. Die Verteilung der besagten Zernike-Koeffizienten ist vorzugsweise eine um die von der Regressionsfunktion gegebenen Mittelwerte zentrierte multivariate Normalverteilung, die für jede Komponente eine unterschiedliche Standardabweichung besitzen kann, und deren Kovarianzmatrix diagonal ist.

Ein solches Modell kann insbesondere aus den von Salmon et al. (z.B. in "Normal-eye Zernike coefficients and root-mean-square wavefront errors", J Catatact Refract Surg, Bd. 32, p. 2064-2074, 2006) veröffentlichten Daten ermittelt werden, wobei linke Augen durch Spiegelung an der Vertikalen in rechte Augen übergeführt wurden, und die Mittelwerte für rechte Augen gelten. Die Zernike-Koeffizienten der radialen Ordnungen 3 bis 5 sind für einen Pupillendurchmesser von 5 mm und eine Akkommodation von 0 dpt angegeben:

$$C_3^{-3} = -0,02359 \pm 0,044 \mu m$$

$$C_3^{-1} = -0,02315 \pm 0,0536 \mu m$$

$$C_3^{1} = -0,00179 \pm 0,0439 \mu m$$

$$C_3^{3} = -0,00001 \pm 0,0352 \mu m$$

$$C_4^{-4} = -0,00223 \pm 0,0153 \mu m$$

$$C_4^{-2} = -0,00323 \pm 0,0107 \mu m$$

$$C_4^{0} = \begin{cases} 0,05052 \pm 0,0321 \mu m & Alter < 45 Jahre \\ 0,05052 \mu m + 0,05 \mu m \cdot (Alter - 45 Jahre)/15 Jahre \pm 0,0321 \mu m & 45 Jahre \leq Alter \leq 60 Jahre \\ 0,10052 \pm 0,0321 \mu m & Alter > 60 Jahre \end{cases}$$

$$C_4^{2} = 0,00114 \pm 0,0184 \mu m$$

$$C_4^{4} = 0,00476 \pm 0,0168 \mu m$$

$$C_5^{-5} = -0,00222 \pm 0,00765 \mu m$$

$$C_5^{-3} = 0,00247 \pm 0,00765 \mu m$$

$$C_5^{-1} = -0,00628 \pm 0,00765 \mu m$$

$$C_5^1 = -0,00021 \pm 0,00612 \mu m$$

$$C_5^3 = -0,00003 \pm 0,00459 \mu m$$

$$C_5^5 = 0,00001 \pm 0,00765 \mu m$$

wobei die Notation $C_n^m = \left\langle C_n^m \right\rangle \pm \sigma_{C_n^m}$ eine abgekürzte Schreibweise für eine Normalverteilung um den Mittelwert $\left\langle C_n^m \right\rangle$ mit gegebener Standardabweichung $\sigma_{C_n^m}$ ist. Im vorliegenden Fall ist die Regressionsfunktion durch den Vektor der Mittelwerte der Zernike-Koeffizienten $\left( \left\langle C_3^{-3} \right\rangle, \left\langle C_3^{-1} \right\rangle, ..., \left\langle C_5^3 \right\rangle, \left\langle C_5^5 \right\rangle \right)$ gegeben, wobei alle Mittelwerte bis auf $\left\langle C_4^0 \right\rangle$ Konstanten sind, und $\left\langle C_4^0 \right\rangle$ vom Alter in der oben beschriebenen Weise abhängt. Die Kovarianzmatrix ist hier eine quadratische Diagonalmatrix mit den quadrierten Standardabweichungen der jeweiligen Zernike-Koeffizienten als Diagonalelemente, $Diag\left( \sigma_{C_3^{-3}}^2, \sigma_{C_3^{-1}}^2, ..., \sigma_{C_5^3}^2, \sigma_{C_5^5}^2 \right)$. Alle Mittelwerte der Zernike-Koeffizienten sind hier konstante Funktionen des Akkommodationszustands, das Modell könnte jedoch problemlos erweitert werden, wenn entsprechende Zernike-Koeffizienten in Abhängigkeit vom Akkommodationszustand des Auges vorliegen.

Schritt B1b: Bereitstellen der Refraktionsdaten eines Auges sowie vorzugsweise sonstiger Daten dieses Auges. Die standardmäßig z.B. durch den Augenoptiker, Ophthalmologen, Optometristen oder ein Gerät erfasste Refraktion (Sphäre, Zylinder, Achse, in der Ferne und/oder Nähe, sowie die Addition) eines Auges wird bereitgestellt und wenn nötig in die Powervektor-Schreibweise umgerechnet. Wenn möglich werden auch sonstige Daten erhoben, wie z.B. Akkommodationszustand des Auges und/oder das Alter des Auges.

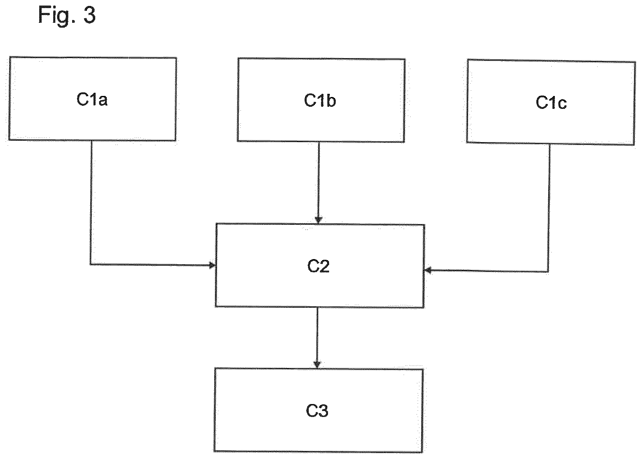

Schritt B1c: Bereitstellen der wahrscheinlichsten Pupillendaten desselben Auges bei mindestens einer Helligkeit. Die wahrscheinlichsten Pupillendaten eines bestimmten Auges darunter Pupillengröße und vorzugsweise Pupillenposition relativ zum Apex werden bereitgestellt. Bevorzugt geschieht das in dem in diesem Dokument dargestellten Verfahren (siehe insbesondere die entsprechenden Details zur Bestimmung der wahrscheinlichsten Pupillendaten eines bestimmten Auges gemäß der weiter unten insbesondere mit Bezug auf Fig. 3 beschriebenen bevorzugten Ausführungsform).

Die wahrscheinlichsten Pupillendaten beruhen soweit möglich auf Messungen des vorliegenden Auges. Sind nicht genügend solcher Messungen vorhanden, oder sind diese zu ungenau, so werden die wahrscheinlichsten Pupillendaten aus der Verteilung der möglichen Pupillendaten ermittelt, die von den Refraktionsdaten und/oder den sonstigen Daten des Auges abhängen können (siehe Hilfsverfahren). Die wahrscheinlichsten Pupillendaten bestehen aus Zahlenwerten (Pupillengröße sowie ggf. Pupillenposition relativ zum Apex) für jede einzelne Helligkeit, oder aber sie liegen als Funktion der Helligkeit vor.

Die Verteilung der möglichen Pupillendaten stellt die als möglich erachteten Pupillendaten dar, wobei die Verteilung soweit möglich auf Messungen des vorliegenden Auges beruht. Die Breite der Verteilung (Standardabweichung) entspricht dabei dem Standardfehler der Messungen. Liegen nicht genügend genaue oder zu wenige Messungen vor, so wird die Verteilung der möglichen Pupillendaten des vorliegenden Auges aus einer Verteilung der Pupillendaten einer Gruppe von Augen ergänzt, welche von den Refraktionsdaten und/oder den sonstigen Daten des Auges abhängen kann (siehe insbesondere die entsprechenden Details zur Bestimmung der wahrscheinlichsten Pupillendaten eines bestimmten Auges gemäß der weiter unten insbesondere mit Bezug auf Fig. 3 beschriebenen bevorzugten Ausführungsform).

Die wahrscheinlichsten Pupillendaten sind jeweils bei mindestens einer Helligkeit gegeben, die später bei der Optimierung des Brillenglases in Schritt B3 benötigt wird, bzw. die während der Refraktion des besagten Auges vorliegt. Insbesondere ist unter mindestens einer Helligkeit ein Kontinuum von Helligkeiten zu verstehen, sodass die wahrscheinlichsten Pupillendaten sowie vorzugsweise die Verteilung der möglichen Pupillendaten bei diesen für jede beliebige Helligkeit angegeben werden können.

Schritt B1d: Gegebenenfalls Bereitstellen mindestens eines funktionalen Zusammenhangs zwischen den Parametern der in B1a bereitgestellten Verteilung und den in B1b bereitgestellten Daten. Sofern im weiteren Schritt (B2) ein statistischer Zusammenhang zwischen einer bestimmten Einflussgröße (z.B. der Addition) und den HOA benötigt wird, es aber nicht möglich ist, einen direkten statistischen Zusammenhang zwischen dieser Einflussgröße und den HOA bereitzustellen, da z.B. keine verbundene Stichprobe dieser Einflussgröße und der HOA existiert, so muss zusätzlich ein funktionaler Zusammenhang zwischen der besagten Einflussgröße und einer anderen Einflussgröße (z.B. dem Alter) bereitgestellt werden, wobei ein direkter statistischer Zusammenhang zwischen der besagten und der anderen Einflussgröße bestehen muss (z.B. zwischen Alter und Addition).

Ein möglicher funktionaler Zusammenhang ist der von Alter und Akkommodationsbreite bei Presbyopen, die sogenannte Duane'sche Kurve. Er ist z.B. Atchison und Smith ("The aging eye", in Optics of the Human Eye, Edinburgh, Elsevier Science Ltd., 200, pp. 221-233) zu entnehmen. Unter Benutzung der gängigen Praxis, die Addition als 2/3 der Akkommodationsbreite zu wählen, ergibt sich ein funktionaler Zusammenhang zwischen Addition und Alter zwischen 45 und 60 Jahren:

$$\text{Alter} = 59.2\,\text{Jahre} + 5.77\ \text{Jahre/dpt} \cdot (\text{Addition} + \text{AN}),$$

wobei AN der Kehrwert des Refraktionsabstands in der Nähe ist, i.d.R. -2.5dpt. Ein weiterer funktionaler Zusammenhang ist der von Akkommodationszustand des Auges und den Refraktionsdaten.

Schritt B2: Rückschließen auf die wahrscheinlichsten Wellenfrontdaten des einen Auges bei mindestens einem Paar aus Pupillendaten und Akkommodationszustand. Falls benötigt, werden zunächst (ein oder mehrere) funktionale Zusammenhänge aus Schritt B1d benutzt, um Daten, die für das Auge in Schritt B1b bereitgestellt wurden, in die Parameter umzurechnen, von denen die in Schritt B1a bereitgestellte Verteilung abhängt. Zum Beispiel muss die in den Refraktionsdaten enthaltene Addition in ein Alter umgerechnet werden, wenn in Schritt B1a eine Verteilung der Pupillendaten in Abhängigkeit vom Alter vorliegt, und in Schritt B1b die in den Refraktionsdaten enthaltene Addition des besagten Auges bereitgestellt wurde.

Als nächstes wird eine bedingte Verteilung der HOA einer Gruppe von Augen berechnet, indem die in B1a bereitgestellte Verteilung der HOA an den in Schritt B1b bereitgestellten Refraktionsdaten des einen Auges und/oder den sonstigen Daten dieses Auges und/oder den in Schritt B1c bereitgestellten wahrscheinlichsten Pupillendaten dieses Auges ausgewertet wird, die gegebenenfalls vorher mit Hilfe der in Schritt B1d bereitgestellten funktionalen Zusammenhänge berechnet wurden. Die bedingte Verteilung der HOA liegt dabei in der Regel für eine Standard-Pupille vor.

Ist die Verteilung der HOA einer Gruppe von Augen als Stichprobe gegeben, so ist die bedingte Verteilung derjenige Teil der Stichprobe, dessen Daten (wahrscheinlichste Pupillendaten und/oder Refraktionsdaten und/oder sonstige Daten) hinreichend nahe an den Daten des einen Auges liegen. Ist die Verteilung der HOA einer Gruppe von Augen als analytische Approximation vorhanden, so berechnet sich die bedingte Verteilung durch Einsetzen der wahrscheinlichsten Pupillendaten und/oder der Refraktionsdaten und/oder der sonstigen Daten des besagten Auges in die analytische Approximation. Ist insbesondere die Verteilung der HOA als Regressionsmodell gegeben, so ergibt sich die bedingte Verteilung der HOA durch einsetzen der Daten des besagten Auges in die Regressionsfunktion sowie in die Kovarianzmatrix.

In analoger Weise ergeben sich die bedingte Verteilung der HOA bei einem oder mehreren Akkommodationszuständen des Auges, welche später in Schritt B3 zur Optimierung benötigt werden, durch Auswerten der bedingen Verteilung der HOAs bei den entsprechenden Akkommodationszuständen. Die wahrscheinlichsten HOA bei den jeweiligen Akkommodationszuständen sind durch das Maximum dieser Verteilung gegeben.

Um die wahrscheinlichsten Wellenfrontdaten bei den gewünschten Akkommodationszuständen und Pupillendaten zu berechnen, müssen zusätzlich zu den jeweiligen HOA auch Aberrationen niederer Ordnung (LOA) ermittelt werden. Dabei werden für die wahrscheinlichsten HOA bei jeweiligem Akkommodationszustand die LOA derart angepasst, dass eine anhand einer geeigneten Metrik aus den LOA, den wahrscheinlichsten HOA und den bei der Refraktion vorliegenden wahrscheinlichsten Pupillendaten berechnete objektive Refraktion identisch mit der in Schritt B1b bereitgestellten subjektiven Refraktion wird.

Die so gewonnenen wahrscheinlichsten Wellenfrontdaten werden schließlich für die Pupillendaten angegeben, die bei der Berechnung oder Optimierung des Brillenglases benötigt werden (Schritt B3). Diese Pupillendaten wurden als wahrscheinlichste Pupillendaten in Schritt B1c bereitgestellt. Dieser Schritt schließt insbesondere das Umskalieren der Zernike-Koeffizienten auf die gegebenen Pupillengrößen ein. Die wahrscheinlichsten Wellenfrontdaten liegen nun für mindestens ein Paar aus Pupillendaten und Akkommodationszustand vor.

Schritt B3: Optimieren eines Brillenglases unter Benutzung der Refraktionsdaten, der wahrscheinlichsten Pupillendaten und der wahrscheinlichsten Wellenfrontdaten. In diesem Schritt werden übliche Verfahren verwendet, um das Brillenglas unter Berücksichtigung der Refraktionsdaten, sowie der Wellenfrontdaten und Pupillendaten zu optimieren. Die Wellenfrontdaten sind dabei durch die in Schritt B2 ermittelten wahrscheinlichsten Wellenfrontdaten gegeben, und die Pupillendaten durch die in Schritt B1c ermittelten wahrscheinlichsten Pupillendaten. Das so optimierte Brillenglas erhöht die Wahrscheinlichkeit einer guten ophthalmischen Versorgung des besagten Auges und verbessert dadurch insgesamt die ophthalmische Versorgung einer Gruppe von Personen.

Eine weitere bevorzugte Ausführungsform der Erfindung bietet ein Verfahren zur Optimierung eines Brillenglases unter Verwendung von wahrscheinlichsten Pupillendaten eines einzelnen Auges. Die Bestimmung der wahrscheinlichsten Pupillendaten eines bestimmten Auges gemäß einer bevorzugten Ausführungsform ist in Fig. 3 schematisch dargestellt. Die in diesem Verfahren bestimmten wahrscheinlichsten Pupillendaten werden vorzugsweise in Schritt B1c weiterverwendet und fließen vorzugsweise letztendlich in die Optimierung des Brillenglases in Schritt B3 ein. Das Verfahren ist vorzugsweise ein Spezialfall des Verfahrens zur Optimierung eines Brillenglases unter Verwendung von wahrscheinlichsten Daten eines einzelnen Auges. Einzelne Schritte dieses bevorzugten Verfahrens gemäß Fig. 3 werden im Folgenden beschrieben:

Schritt C1a: Bereitstellen einer Verteilung der Pupillendaten einer Gruppe von Augen, die von den Parametern Helligkeit, sowie vorzugsweise Refraktionsdaten und/oder sonstigen Daten abhängt. Vorzugsweise wird zunächst eine Verteilung der Pupillendaten einer Gruppe von Augen bereitgestellt, die von den Parametern Helligkeit, und wenn möglich auch von den Refraktionsdaten und/oder sonstigen Daten abhängt. Diese Verteilung kann analog zu Schritt B1a als eine Stichprobe oder in Form eines analytischen Modells vorliegen. Die Verteilung stellt die für eine Gruppe von Augen bekannte statistische Information über die Pupillendaten und die Refraktionsdaten und/oder die sonstigen Daten einer Gruppe von Augen dar.

Ein mögliches analytisches Modell einer solchen Verteilung besteht aus einer vektorwertigen Regressionsfunktion und einem multivariat normalverteilten Regressionsfehler (vgl. Abschnitt über analytisches Modell der Verteilung von Zernike-Koeffizienten in Schritt B1a). Sowohl die Regressionsfunktion als auch die Kovarianzmatrix des Regressionsfehlers ist dabei eine vektor- bzw. matrixwertige Funktion mindestens eines der folgenden Parameter: der Refraktion in Powervektor-Darstellung, der Addition, dem Alter, dem Akkommodationszustand, und insbesondere der Helligkeit bzw. des Logarithmus der Helligkeit. Die Regressionsfunktion und Kovarianzmatrix erhält man vorzugsweiseweise durch minimieren der mit der Kovarianzmatrix gewichteten Fehlerquadrate einer Stichprobe. Jede Komponente der Regressionsfunktion kann in der Form eines abschnittsweise definierten Polynoms der oben genannten Parameter vorliegen, und gibt die im Mittel vorhandene Abhängigkeit der jeweiligen dieser Komponente zugeordneten Pupillendaten von den Parametern an, z.B. die Abhängigkeit der Pupillengröße von der Helligkeit und/oder die Abhängigkeit der Pupillenposition relativ zum Apex von der Helligkeit. Insbesondere kann eine solche Funktion abschnittsweise eine konstante Funktion, eine lineare Funktion oder eine quadratische Funktion der Parameter sein.

Der multivariat normalverteilte Regressionsfehler kann, muss aber nicht, eine nichtdiagonale Kovarianzmatrix besitzen, die ebenfalls eine Funktion der oben genannten Parameter sein kann. Eine solche Funktion (d.h. jedes Element der Kovarianzmatrix) kann wie die Regressionsfunktion selbst als abschnittsweise konstante Funktion, lineare Funktion oder eine quadratische Funktion der Parameter genähert sein. Hängt insbesondere der Regressionsfehler nicht von den Parametern ab, so ist die Kovarianzmatrix eine konstante Funktion der Parameter. Ergeben sich keine statistischen Zusammenhänge zwischen einzelnen Komponenten der Pupillendaten, so ist die Kovarianzmatrix diagonal.

Ein Spezialfall des oben beschriebenen analytischen Modells ist die Abhängigkeit einer einzigen Pupillengröße (hier betrachtet als Pupillendurchmesser) von Alter und Helligkeit, und wurde der Publikation von Winn et al. entnommen. Die Regressionsfunktion $\overline{d}(E_V, a)$ gibt den Pupillendurchmesser als Funktion des Alters $a$ und der Beleuchtungsstärke in der Pupillenebene $E_V$ in Lux an. Mit letzterer wird die mit dem Auge beobachtete Helligkeit modelliert.

$$\overline{d}(E_V, a) = 8{,}95\,mm - 1{,}557\,mm \cdot \log_{10} E_V - 0{,}0509\,\frac{mm}{Jahr} \cdot a + 0{,}0110\,\frac{mm}{Jahr} \cdot a \log_{10} E_V$$

Der Standardfehler, und damit die Standardabweichung der Verteilung der Pupillendurchmesser beträgt unabhängig von Alter und Beleuchtungsstärke $\sigma_d = 1{,}0\,mm$. Insgesamt ist die Verteilung der Pupillendurchmesser also durch

$$\overline{d}(E_V, a) \sim Normal\left(\overline{d}(E_V, a), \sigma_d^2\right)$$

gegeben, wobei $Normal(x,y)$ die Normalverteilung mit Erwartungswert $x$ und Varianz $y$ ist.

Ein anderer Spezialfall betrifft die Verteilung der Änderung $\partial d / \partial \log_{10} E_V$ des Pupillendurchmessers $d$ pro logarithmischer Beleuchtungsdichte $\log_{10} E_V$ in Abhängigkeit vom Alter. Die Verteilung ist nach Winn et al. durch

$$\frac{\partial d}{\partial \log_{10} E_V} \sim Normal\left(PV, \sigma_{PV}^2\right)$$

mit

$$PV = -0{,}11 \frac{mm}{Jahr \cdot \log_{10} lux} \times Alter + 1{,}557 \frac{mm}{\log_{10} lux}$$

$\sigma_{PV}^2 = 0{,}2 \dfrac{mm}{\log_{10} lux}$ und gegeben ist.

Schritt C1 b: Bereitstellen von Refraktionsdaten eines Auges und/oder von sonstigen Daten desselben Auges, sowie vorzugsweise von Pupillendaten desselben Auges bei mindestens einer Helligkeit. Vorzugsweise verläuft dieser Schritt analog zu Schritt B1b, was die Bereitstellung von Refraktionsdaten eines Auges und/oder sonstiger Daten des Auges betrifft. Wenn möglich, werden ebenfalls Pupillendaten des Auges bei mindestens einer Helligkeit bereitgestellt, was z.B. durch Messung mit einem Gerät oder eine manuelle Messung geschehen kann.

Schritt C1c: Gegebenenfalls Bereitstellen mindestens eines funktionalen Zusammenhangs zwischen den Parametern der in C1a bereitgestellten Verteilung und den in C1b bereitgestellten Daten. Vorzugsweise werden wie in Schritt B1d wenn nötig funktionale Zusammenhänge zwischen den in C1c bereitgestellten Parametern der Verteilung, die keine Pupillendaten sind, und den in Schritt C1b verwendeten Daten, die keine Refraktionsdaten sind, hergestellt. Ein möglicher funktionaler Zusammenhang ist der in Schritt B1d genannte Zusammenhang zwischen Alter und Addition.

Schritt C2. Rückschließen auf die wahrscheinlichsten Pupillendaten des einen Auges bei mindestens einer Helligkeit. Es wird auf die wahrscheinlichsten Pupillendaten des besagten Auges bei mindestens einer Helligkeit geschlossen. Die Helligkeit bzw. die Helligkeiten sind dabei zumindest die bei der Refraktion vorliegende Helligkeit, sowie weitere Helligkeiten, die bei der Optimierung des Brillenglases in Schritt B3 benötigt werden. Falls benötigt, werden zunächst (ein oder mehrere) funktionale Zusammenhänge aus Schritt C1c benutzt, um Daten, die für das Auge in Schritt C1b bereitgestellt wurden, in die Parameter umzurechnen, von denen die in Schritt C1a bereitgestellte Verteilung abhängt. Zum Beispiel kann so die in den Refraktionsdaten enthaltene Addition in ein Alter umgerechnet werden, wenn in Schritt C1a eine Verteilung der Pupillendaten und des Alters vorliegt, und in Schritt C1b die in den Refraktionsdaten enthaltene Addition des besagten Auges bereitgestellt wurde.

Falls in C1b keine Pupillendaten oder Pupillendaten bei nur einer Helligkeit bereitgestellt wurden, wird zunächst eine bedingte Verteilung der helligkeitsabhängigen Pupillendaten einer Gruppe von Augen berechnet, indem die in C1a bereitgestellte Verteilung bei gegebenen Refraktionsdaten und/oder sonstigen Daten des einen Auges ausgewertet wird. Die Refraktionsdaten und/oder sonstigen Daten des einen Auges können dabei gegebenenfalls auch mit Hilfe der in C1c bereitgestellten funktionalen Zusammenhänge berechnet worden sein.

Ist die Verteilung der helligkeitsabhängigen Pupillendaten einer Gruppe von Augen als Stichprobe gegeben, so ist die bedingte Verteilung derjenige Teil der Stichprobe, der hinsichtlich auf die Refraktionsdaten und/oder die sonstigen Daten sich hinreichend wenig von den Refraktionsdaten und/oder den sonstigen Daten des einen Auges unterscheidet. Ist die Verteilung der helligkeitsabhängigen Pupillendaten einer Gruppe von Augen als analytische Approximation vorhanden, so berechnet sich die bedingte Verteilung durch Einsetzen der Refraktionsdaten und/oder der sonstigen Daten des besagten Auges in die analytische Approximation.

Wurden in C1b keine Pupillendaten des besagten Auges bereitgestellt, dann sind die wahrscheinlichsten Pupillendaten identisch mit dem Maximum der bedingten Verteilung der Pupillendaten ausgewertet bei den entsprechenden Helligkeiten. Ist die Verteilung insbesondere als analytische Approximation gegeben, die aus einer Regressionsfunktion und einem normalverteilten Regressionsfehler besteht, so sind die wahrscheinlichsten Pupillendaten durch den Wert der Regressionsfunktion gegeben, in die die Refraktionsdaten und/oder sonstigen Daten des besagten Auges und die entsprechenden Helligkeiten eingesetzt wurden.

Wurden in C1b jedoch Pupillendaten des besagten Auges bereitgestellt, so können folgende Fälle unterschieden werden:

Fall 1: Bereitstellen von Pupillendaten bei zwei oder mehr Helligkeiten. In diesem Fall sind die wahrscheinlichsten Pupillendaten entweder die bereitgestellten Pupillendaten, oder aber, falls die wahrscheinlichsten Pupillendaten bei anderen Helligkeiten ermittelt werden sollen, werden diese durch Interpolation und/oder Extrapolation der bereitgestellten Pupillendaten berechnet. Dabei werden die vom Logarithmus der Helligkeit abhängigen Pupillendaten linear oder mittels Splines interpoliert bzw. extrapoliert. Da die in Schritt C1a bereitgestellte Verteilung viel weniger Information über die Pupillendaten des besagten Auges enthält als die bereitgestellten Pupillendaten für dieses Auge, geht die bedingte Verteilung der Pupillendaten in diesem Fall nicht in die Berechnung der wahrscheinlichsten Pupillendaten des besagten Auges ein.

Fall 2: Bereitstellen von Pupillendaten bei einer einzigen Helligkeit. Um von den bereitgestellten Pupillendaten auf die Pupillendaten bei anderen Helligkeiten zu schließen, wird die bedingte Verteilung der helligkeitsabhängigen Pupillendaten einer Gruppe von Augen zusammen mit den bereitgestellten Pupillendaten verwendet. Dabei wird die bedingte Verteilung an den für das besagte Auge bereitgestellten Pupillendaten ausgewertet, sodass sich eine weitere bedingte Verteilung der Pupillendaten bei mindestens einer Helligkeit ergibt. Die wahrscheinlichsten Pupillendaten bei mindestens einer Helligkeit sind gegeben durch das Maximum der weiteren bedingten Verteilung bei den entsprechenden Helligkeiten.

Schritt C3. Verwenden der in C2 bestimmten wahrscheinlichsten Pupillendaten des einen Auges zur Berechnung eines Brillenglases durch Bereitstellen in Schritt B1c. Die so bestimmten wahrscheinlichsten Pupillendaten des besagten Auges bei mindestens einer Helligkeit werden verwendet, um sie in Schritt B1c bereitzustellen. Sie fließen dadurch in die Optimierung des Brillenglases in Schritt B3 ein.

**Patentansprüche**

1.  Verfahren zum Optimieren und Herstellen eines individuellen Brillenglases für zumindest ein Auge eines Brillenträgers, umfassend:

    - Bereitstellen (A1a; B1a) einer Verteilung von Daten für eine Vielzahl von Augen umfassend ein Bereitstellen (B1a) einer Verteilung von Abbildungsfehlern höherer Ordnung der Vielzahl von Augen, welche sich zumindest teilweise in zumindest einem weiteren physikalischen und/oder physiologischen Parameter, darunter zumindest in Pupillendaten, unterscheiden;
    - Bestimmen (A1b, A1c, A1d; B1b, B1c, B1d) eines Wertes des zumindest einen weiteren physikalischen und/oder physiologischen Parameters für das zumindest eine Auge des Brillenträgers, umfassend ein Bereitstellen (B1c) von wahrscheinlichsten Pupillendaten, einschließlich der Pupillengröße, für das zumindest eine Auge des Brillenträgers bei zumindest einer Helligkeit, für die das Brillenglas optimiert und hergestellt werden soll;
    - Bestimmen (A2; B2) der gemäß der bereitgestellten Verteilung von Daten wahrscheinlichsten Daten, WD2, des zumindest einen Auges des Benutzers bei dem bestimmten Wert des zumindest einen weiteren physikalischen und/oder physiologischen Parameters für das zumindest eine Auge, umfassend ein Bestimmen (B2) der gemäß der bereitgestellten Verteilung von Abbildungsfehlern höherer Ordnung wahrscheinlichsten Werte für die Abbildungsfehler höherer Ordnung des zumindest einen Auges des Benutzers bei dem bestimmten Wert des zumindest einen weiteren physikalischen und/oder physiologischen Parameters für das zumindest eine Auge,
    wobei das Brillenglas unter Berücksichtigung der bestimmten wahrscheinlichsten Daten WD2 optimiert wird, wobei das Brillenglas derart optimiert wird, dass es die Abbildungsfehler höherer Ordnung des zumindest einen Auges der Benutzers gemäß deren bestimmten wahrscheinlichsten Werten berücksichtigt bzw. zumindest teilweise korrigiert.

2.  Verfahren nach Anspruch 1, wobei das Bestimmen der wahrscheinlichsten Daten des zumindest einen Auges des Benutzers ein Berechnen einer bedingten Verteilung der Daten der Vielzahl von Augen umfasst, indem die bereitgestellte Verteilung der Daten der Vielzahl von Augen an dem für das zumindest eine Auge bestimmten Wert des Parameters ausgewertet wird, wobei vorzugsweise die wahrscheinlichsten Daten des zumindest einen Auges durch die Maxima der berechneten bedingten Verteilung gegeben sind.

3.  Verfahren nach Anspruch 1 oder 2,

    wobei die Verteilung von Abbildungsfehlern höherer Ordnung der Vielzahl von Augen derart bereitgestellt wird, dass sich die Augen zumindest teilweise in Refraktionsdaten unterscheiden; und
    wobei das Bestimmen eines Wertes des zumindest einen weiteren physikalischen und/oder physiologischen Parameters für das zumindest eine Auge des Brillenträgers ein Bereitstellen (B1b) von Werten der Refraktionsdaten für das zumindest eine Auges des Brillenträgers umfasst.

4. Verfahren nach einem der Ansprüche 1 bis 3, wobei das Bereitstellen (B1c) von wahrscheinlichsten Pupillendaten durch Messung an dem zumindest einen Auge des Brillenträgers erfolgt.

5. Verfahren nach einem der Ansprüche 1 bis 3, wobei das Bereitstellen (B1c) von wahrscheinlichsten Pupillendaten des Auges im Rahmen des Bestimmens eines Wertes des zumindest einen weiteren physikalischen und/oder physiologischen Parameters für das zumindest eine Auge des Brillenträgers umfasst:

   - Bereitstellen (C1a) einer Verteilung von Pupillendaten einer Gruppe von Augen, die von den Parametern Helligkeit und Refraktionsdaten abhängen;
   - Bereitstellen (C1b) von Refraktionsdaten des zumindest einen Auges des Brillenträgers bei zumindest einer Helligkeit;
   - Rückschließen (C2) auf die wahrscheinlichsten Pupillendaten bei einer Helligkeit, für die das Brillenglas optimiert und hergestellt werden soll.

6. Verfahren nach Anspruch 5, wobei das Rückschließen (C2) auf die wahrscheinlichsten Pupillendaten ein Berechnen einer bedingten Verteilung der Pupillendaten der Gruppe von Augen umfasst, indem die bereitgestellte Verteilung von Pupillendaten der Gruppe von Augen bei den bereitgestellten Refraktionsdaten des zumindest einen Auges ausgewertet wird.

7. Verfahren nach einem der vorangegangenen Ansprüche, wobei das Bestimmen eines Wertes des zumindest einen weiteren physikalischen und/oder physiologischen Parameters für das zumindest eine Auge des Brillenträgers ein Bereitstellen von Refraktionsdaten des Auges, insbesondere von Werten für Sphäre, Zylinder, Achse, in der Ferne und/oder Nähe, sowie der Addition, umfasst.

8. Verfahren nach einem der vorangegangenen Ansprüche, wobei das Bestimmen eines Wertes des zumindest einen weiteren physikalischen und/oder physiologischen Parameters für das zumindest eine Auge des Brillenträgers ein Bereitstellen (A1d; B1d) zumindest eines funktionalen Zusammenhangs zwischen einem für das zumindest eine Auge bereitgestellten Parameter und dem zumindest einen weiteren physikalischen und/oder physiologischen Parameter umfasst.

9. Verfahren nach Anspruch 8, wobei der zumindest eine funktionale Zusammenhang einen funktionalen Zusammenhang zwischen Alter und Addition umfasst.

10. Verfahren nach Anspruch 8 oder 9, wobei der zumindest eine funktionale Zusammenhang einen funktionalen Zusammenhang zwischen Akkommodationszustand und Refraktionsdaten des Auges umfasst.


**Claims**

1. A method of optimising and producing an individual spectacle lens for at least one eye of a spectacle wearer, comprising:

   - providing (A1a; B1a) a distribution of data for a plurality of eyes comprising providing (B1a) a distribution of higher order aberrations of a plurality of eyes which differ at least partially in at least one further physical and/or physiological parameter;
   - determining (A1b, A1c, A1d; B1b, B1c, B1d) a value of said at least one further physical and/or physiological parameter for said at least one eye of said spectacle wearer comprising providing (B1c) most likely pupil data, including pupil size, for said at least one eye of said spectacle wearer at at least one brightness for which said spectacle lens is to be optimised and manufactured;
   - determining (A2; B2) the most likely data, WD2, of the at least one eye of the user at the determined value of the at least one further physical and/or physiological parameter for the at least one eye according to the provided distribution of data, comprising determining (B2) the most likely values for the higher order aberrations of the at least one eye of the user at the determined value of the at least one further physical and/or physiological parameter for the at least one eye according to the provided distribution of higher order aberrations,

   wherein the spectacle lens is optimised taking into account the determined most probable data WD2, wherein the spectacle lens is optimised such that it takes into account or at least partially corrects the higher order aberrations of the at least one eye of the user according to their determined most probable values.

**2.** Method according to claim 1, wherein determining the most likely data of the at least one eye of the user comprises calculating a conditional distribution of the data of the plurality of eyes by evaluating the provided distribution of the data of the plurality of eyes at the value of the parameter determined for the at least one eye, preferably wherein the most likely data of the at least one eye is given by the maxima of the calculated conditional distribution.

**3.** Method according to claim 1 or 2,

wherein the distribution of higher order aberrations of the plurality of eyes is provided such that the eyes differ at least in part in refraction data; and
wherein determining a value of the at least one further physical and/or physiological parameter for the at least one eye of the spectacle wearer comprises providing (B1b) values of the refraction data for the at least one eye of the spectacle wearer.

**4.** Method according to any one of claims 1 to 3,
wherein providing (B1c) most likely pupil data by measurement on the at least one eye of the spectacle wearer.

**5.** Method according to any one of claims 1 to 3,
wherein providing (B1c) most likely pupil data of the eye comprises in the course of determining a value of the at least one further physical and/or physiological parameter for the at least one eye of the spectacle wearer:

- providing (C1a) a distribution of pupil data of a group of eyes depending on the parameters brightness and refraction data;
- providing (C1b) refraction data of the at least one eye of the spectacle wearer at at least one brightness;
- inferring (C2) the most likely pupil data at a brightness for which the spectacle lens is to be optimised and manufactured.

**6.** Method according to claim 5, wherein inferring (C2) the most likely pupil data comprises calculating a conditional distribution of the pupil data of the set of eyes by evaluating the provided distribution of pupil data of the set of eyes at the provided refraction data of the at least one eye.

**7.** Method according to any one of the preceding claims, wherein determining a value of the at least one further physical and/or physiological parameter for the at least one eye of the spectacle wearer comprises providing refraction data of the eye, in particular values for sphere, cylinder, axis, at distance and/or near, and addition.

**8.** Method according to any one of the preceding claims, wherein determining a value of the at least one further physical and/or physiological parameter for the at least one eye of the spectacle wearer comprises providing (A1d; B1d) at least one functional relationship between a parameter provided for the at least one eye and the at least one further physical and/or physiological parameter.

**9.** Method according to claim 8, wherein the at least one functional relationship comprises a functional relationship between age and addition.

**10.** Method according to claim 8 or 9, wherein the at least one functional relationship comprises a functional relationship between accommodation state and refraction data of the eye.

**Revendications**

**1.** Procédé d'optimisation et de fabrication d'un verre de lunettes individuel pour au moins un oeil d'un porteur de lunettes, comprenant :

- fournir (A1a ; B1a) une distribution de données pour une pluralité d'yeux comprenant une fourniture (B1a) d'une distribution d'erreurs d'imagerie d'ordre supérieur d'une pluralité d'yeux qui diffèrent au moins partiellement dans au moins un autre paramètre physique et/ou physiologique ;
- déterminer (A1b, A1c, A1d ; B1b, B1c, B1d) une valeur dudit au moins un autre paramètre physique et/ou physiologique pour ledit au moins un oeil du porteur de lunettes, comprenant la fourniture (B1c) de données de pupille les plus probables, incluant la taille de pupille, pour ledit au moins un oeil du porteur de lunettes à au moins une luminosité pour laquelle le verre de lunettes doit être optimisé et fabriqué ;

- déterminer (A2 ; B2) les données les plus probables WD2, selon la distribution de données fournie, de l'au moins un oeil de l'utilisateur à la valeur déterminée de l'au moins un autre paramètre physique et/ou physiologique pour l'au moins un oeil, comprenant une détermination (B2) des valeurs les plus probables, selon la distribution de défauts d'imagerie d'ordre supérieur fournie, pour les défauts d'imagerie d'ordre supérieur de l'au moins un oeil de l'utilisateur à la valeur déterminée de l'au moins un autre paramètre physique et/ou physiologique pour l'au moins un oeil,

le verre de lunettes étant optimisé en tenant compte des données déterminées les plus probables WD2, le verre de lunettes étant optimisé de telle sorte qu'il tienne compte ou corrige au moins partiellement les erreurs de représentation d'ordre supérieur de l'au moins un oeil de l'utilisateur selon leurs valeurs déterminées les plus probables.

2. Procédé selon la revendication 1, dans lequel la détermination des données les plus probables de l'au moins un oeil de l'utilisateur comprend le calcul d'une distribution conditionnelle des données de la pluralité d'yeux en évaluant la distribution fournie des données de la pluralité d'yeux à la valeur du paramètre déterminée pour l'au moins un oeil, de préférence les données les plus probables de l'au moins un oeil étant données par les maxima de la distribution conditionnelle calculée.

3. Procédé selon la revendication 1 ou 2,

dans lequel la distribution des erreurs d'imagerie d'ordre supérieur de la pluralité d'yeux est fournie de telle sorte que les yeux diffèrent au moins partiellement en données de réfraction ; et
dans lequel la détermination d'une valeur dudit au moins un autre paramètre physique et/ou physiologique pour ledit au moins un oeil du porteur de lunettes comprend la fourniture (B1b) de valeurs des données de réfraction pour ledit au moins un oeil du porteur de lunettes.

4. Procédé selon l'une quelconque des revendications 1 à 3,
dans lequel la fourniture (B1c) de données de pupille les plus probables est effectuée par mesure sur l'au moins un oeil du porteur de lunettes.

5. Procédé selon l'une des revendications 1 à 3,
dans lequel la fourniture (B1c) de données de pupille les plus probables de l'œil comprend, dans le cadre de la détermination d'une valeur dudit au moins un autre paramètre physique et/ou physiologique pour ledit au moins un oeil du porteur de lunettes :

- fournir (C1a) une distribution de données de pupille d'un groupe d'yeux qui dépendent des paramètres de luminosité et de données de réfraction ;
- fournir (C1b) des données de réfraction de l'au moins un oeil du porteur de lunettes à au moins une luminosité ;
- inférer (C2) les données de pupille les plus probables à une luminosité pour laquelle le verre de lunettes doit être optimisé et fabriqué.

6. Procédé selon la revendication 5, dans lequel l'inférence (C2) des données de pupille les plus probables comprend le calcul d'une distribution conditionnelle des données de pupille du groupe d'yeux en évaluant la distribution fournie de données de pupille du groupe d'yeux dans les données de réfraction fournies dudit au moins un oeil.

7. Procédé selon l'une quelconque des revendications précédentes, dans lequel la détermination d'une valeur dudit au moins un autre paramètre physique et/ou physiologique pour ledit au moins un oeil du porteur de lunettes comprend la fourniture de données de réfraction de l'oeil, en particulier des valeurs de sphère, de cylindre, d'axe, en vision de loin et/ou de près, ainsi que l'addition.

8. Procédé selon l'une quelconque des revendications précédentes, dans lequel la détermination d'une valeur dudit au moins un autre paramètre physique et/ou physiologique pour ledit au moins un oeil du porteur de lunettes comprend la fourniture (A1d ; B1d) d'au moins une relation fonctionnelle entre un paramètre fourni pour ledit au moins un oeil et ledit au moins un autre paramètre physique et/ou physiologique.

9. Procédé selon la revendication 8, dans lequel ladite au moins une relation fonctionnelle comprend une relation fonctionnelle entre l'âge et l'addition.

10. Procédé selon la revendication 8 ou 9, dans lequel ladite au moins une relation fonctionnelle comprend une relation fonctionnelle entre l'état d'accommodation et les données de réfraction de l'oeil.

Fig. 1

Fig. 2

Fig. 3

**EP 3 785 601 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10313275
- WO 2004072709 A1
- US 2011001925 A1
- WO 2006116820 A1

**In der Beschreibung aufgeführte Nicht-Patentliteratur**

- Normal-eye Zernike coefficients and root-mean-square wavefront errors. *J Catatact Refract Surg,* 2006, vol. 32, 2064-2074
- The aging eye. **ATCHISON ; SMITH.** Optics of the Human Eye. Elsevier Science Ltd, vol. 200, 221-233